# EUROPEAN PATENT SPECIFICATION

(11) **EP 2 185 126 B1**
(45) Date of publication and mention of the grant of the patent: **21.06.2017**
(21) Application number: 07802528.5
(22) Date of filing: 07.08.2007
(51) Int. Cl.: A61K 8/49, A61K 8/60, A61Q 17/04

(54) **USE OF TROXERUTIN TO QUENCH THE FLUORESCENCE OF DISODIUM PHENYL DIBENZIMIDAZOLE TETRASULFONATE**
VERWENDUNG VON TROXERUTIN ZUR DÄMPFUNG DER FLUORESZENZ VON DINATRIUMHENYLDIBENZIMIDAZOL-TETRASULFONAT
UTILISATION DE TROXÉRUTINE POUR PIÉGER LA FLUORESCENCE DU DISODIUM PHÉNYL DIBENZIMIDAZOLE TETRASULFONATE

(43) Date of publication of application: 19.05.2010
(73) Proprietor: Symrise AG, 37603 Holzminden (DE)
(72) Inventor: JOHNCOCK, William, 21465 Reinbek (DE); CLAUS, Jürgen, 37639 Bevern (DE)
(74) Representative: Fabry, Bernd
(86) International application number: PCT/EP2007/058208
(87) International publication number: WO 2007/128840

(56) References cited:
- DE-A1- 19 755 504
- DE-A1- 19 923 712
- US-A1- 2006 257 338

## Description

The present invention relates to cosmetic preparations for protection of the human skin and human hair against the harmful effects of ultraviolet solar radiation containing the water soluble UVA absorbing substance disodium phenyl dibenzimidazole tetrasulfonate [2,2'-(1,4-Phenylene)bis(1H-benzimidazole-4,6-disulfonic acid, monosodium salt); 1H-Benzimidazole-4,6-Disulfonic Acid, 2,2'-(1,4-Phenylene)Bis-, Disodium Salt] together with troxerutin (7,3',4'-tris[O-(2-hydroxyethyl)]rutin = 3,5-dihydroxy-3',4',7-tris(2-hydroxyethoxy)flavone 3-(6-O-(6-deoxy-alpha-L-mannopyranosyl)-beta-D-glucopyranoside) to quench the fluorescence of disodium phenyl dibenzimidazole tetrasulfonate, and corresponding methods of manufacturing such preparations and uses of troxerutin and disodium phenyl dibenzimidazole tetrasulfonate. In this description and claims, no differentiation is made between disodium phenyl dibenzimidazole tetrasulfonic acid and any salts thereof, unless otherwise indicated. Thus, the terms disodium phenyl dibenzimidazole tetrasulfonate, its salts and disodium phenyl dibenzimidazole tetrasulfonic acid and its salts are used interchangeably unless indicated otherwise.

UV absorbers are compounds which have a pronounced absorption capacity for ultraviolet radiation. They are used in particular as sunscreens in cosmetic and pharmaceutical preparations, but also to improve the light fastness of industrial products, such as paints, varnishes, plastics, textiles, polymers such as, for example, polymers and copolymers of mono- and diolefins, polystyrenes, polyurethanes, polyamides, polyesters, polyureas and polycarbonates, packaging materials and rubbers.

UV rays are classified according to wavelength as UVA rays (320-400 nm, UVA-I: 340-400 nm, UVA-II: 320-340 nm) or UVB rays (280-320 nm). UV rays can cause acute and chronic damage to the skin, the type of damage depending on the wavelength of the radiation. For instance, UVB radiation can cause sunburn (erythema) extending to most severe burning of the skin; reduction in enzyme activities, weakening of the immune system, disturbances of the DNA structure and changes in the cell membrane are also known as harmful effects of UVB rays. UVA rays penetrate into deeper layers of the skin where they can accelerate the aging process of the skin. The shorter wave UVA-II radiation additionally contributes to the development of sunburn. Moreover, UVA radiation can trigger phototoxic or photoallergic skin reactions. Very frequent and unprotected irradiation of the skin by sunlight leads to a loss of skin elasticity and to increased development of wrinkles. In extreme cases, pathogenic changes in the skin extending to skin cancer are observed. To attenuate these negative effects of UV radiation, materials which absorb or reflect UV light, generally called UV absorbers, are used in cosmetic or pharamacological preparations. The UV absorbers are classified as UVA and UVB absorbers depending on the location of their absorption maxima; if a UV absorber absorbs both UVA and UVB, it is referred to as a UVA/B broadband absorber.

The number of suitable UVA absorbers is very limited and they have considerable deficiencies. Thus, the filter 4-tert.-butyl-4'-methoxy-dibenzoylmethane (Butyl methoxydibenzoylmethane; absorption maximum at 357 nm) that is frequently used in particular for protection against UVA I radiation is not photostable. Photoreactions are also observed in combination with the UVB filters 2-ethylhexyl and isoamyl p-methoxycinnamate. Moreover, it has only a limited solubility in cosmetic oils, which can lead to problems in the formulation of cosmetic preparations. Furthermore, sunscreen products containing dibenzoylmethane derivatives can leave marks on textiles that are extremely difficult to wash out.

A relatively novel UVA filter that does not have these disadvantages is disodium phenyl dibenzimidazole tetrasulfonate sold under the trade name of Neo Heliopan® AP (c.f. e.g. EP 669 323, WO 03/084496; Symrise GmbH & Co. KG, Germany). This very photostable UVA filter harmlessly dissipates its absorbed UV energy via fluorescence/phosphorescence which by a small number of consumers is regarded as a negative. There is therefore a need for agents to quench this fluorescence for use in cosmetic and pharmaceutical preparations. The quencher should not be noticably coloured, and should be stable in typical cosmetical and dermal formulations, particularly in sunscreens, without causing discoloration. The quencher should also allow for an easy incorporation in typical cosmetical and/or dermatological formulations, particularly in sunscreens, and thus should preferably be water soluble in such concentrations as are necessary for quenching of disodium phenyl dibenzimidazole tetrasulfonic acid and/or its salts.

It is known that quenching of fluorescence can occur by a number of mechanisms including self quenching when high concentrations of the substance are present; static quenching which occurs from a complex formed by the ground state of the fluorescor and the quencher; and colour quenching in which coloured molecules absorb the emitted fluorescence energy. Typical quenchers include oxygen, but singlet oxygen is produced as a result and so is undesirable in cosmetics and pharmaceutical preparations; heavy metals such as thallium, or certain organic compounds such as alkyl bromide/fluoride/chlorides, tetrafluoroborates, anthracene derivatives, could in principle be used but are toxic and so are of no use in cosmetics and pharmaceutical preparations; and colours, which will give an unacceptable colour to cosmetics and pharmaceutical preparations. Additional possible quenchers are water soluble plant extracts which contain UV and visible light absorbing chromophors such as but not restricted to Green Tea, Rooibos, *Sophora japonica,*or the UV absorbing materials that are found in the plant extracts such as rutin, riboflavins, tannins, nictinamides, quercetin, ubiquinones, polyphenols etc, or their derivatives. However, these materials which are potent antioxidants are either unstable in cosmetic preparations on storage or can lead to unacceptable colouration of the preparations either directly on application or within a short period of time at ambient temperatures. This discolouration is accelerated at temperatures of 30°C or more, which for cosmetic formulations, especially suncare products, is even more unacceptable.

The flavone derivative rutin or its aglycon, quercetin, are both effective quenchers of the fluorescence of disodium phenyl dibenzimidazole tetrasulfonate and its salts. However an emulsion prepared with quercetin is deeply yellow in colour and is not cosmetically acceptable. An emulsion prepared with rutin is beige in colour and on storage turns dark brown which is also not cosmetically acceptable. This colour instability is typical of flavone derivatives such as kaempferol, luteolion, fisetin, apigenin, morin, robinetin, gossypetin, myricetin or their sugar derivatives such as quercitrin, monoxerutin, diosmin, etc. The flavone sugar derivatives naringin and hesperidin, though less susceptible to colouration problems than other flavone derivatives, do not effectively quench the fluorescence of disodium phenyl dibenzimidazole tetrasulfonate and its salts. They are also poorly soluble in water.

It was therefore very surprising and unexpected that the flavone derivative troxerutin exhibited long term colour stability in an emulsion while simultaneously being an effective quencher of the fluorescence of disodium phenyl dibenzimidazole tetrasulfonate. A further advantage of using troxerutin is its ease of solubility in water compared to other derivatives such as naringin and hesperidin.

The use of troxerutin in cometic formulations as such is not new. For example DE 197 39 349 teaches the use of troxerutin as an antioxidant and free radical scavenger in cosmetic formulations to protect the skin from oxidative processes and to protect the cosmetic preparation itself. It does not teach its use as a fluorescence quencher in cosmetic formulations.

EP 1 055 413 (cf. DE 199 23 712) teaches the use of flavone derivatives, including troxerutin, together with sulfonated UV filters to improve the protection against UV induced damage. It does not teach the use of troxerutin to quench the fluorescence of disodium phenyl dibenzimidazole tetrasulfonate or its salts.

US 4,603,046 teaches the use of troxerutin together with UV filters, particularly ethylhexyl p-dimethylaminobenzoate to improve the protection against erythema. It does not mention any fluorescent quenching properties of troxerutin.

DE 197 55 504 teaches the use of flavone derivatives including troxerutin, to stabilise the photolabile UVA filter butyl methoxydibenzoylmethane. It does not teach about quenching fluorescence.

DE 10 2006 030 328 teaches the use of flavones including troxerutin together with trioses and/or tetroses and the optional use of UV filters to improve the skin lightening properties of a formulation. It does not teach anything about fluorescence quenching.

According to the invention, there is thus provided the use of troxerutin to quench the fluorescence of disodium phenyl dibenzimidazole tetrasulfonic acid and/or one or more salts thereof. Preferably, the use is in a cosmetic or pharmaceutical, especially dermatological, preparation, and particularly preferably a sunscreen formulation.

Preferably, the total amount of troxerutin for use in a cosmetic or pharmaceutical, especially dermatological, preparation is in the range of 0.1 to 2 wt.-%, even more preferred in the range of 0.3 to 1 wt.-%, based on the total weight of the preparation; simultaneously, the total content of disodium phenyl dibenzimidazole tetrasulfonic acid and its salts is preferably in the range of 0.2 to 4 wt.-%, even more preferably in the range of 0.3 to 3 wt.-%, based on the total weight of the preparation. Thus, the weight ratio of disodium phenyl dibenzimidazole tetrasulfonic acid and its salts to troxerutin is 40:1 to 1:10, preferably 10:1 to 1:4. A preparation (particularly a cosmetic and/or pharmaceutical preparation) may comprise troxerutin and disodium phenyl dibenzimidazole tetrasulfonate and/or one or more salts thereof in combination with one or more further UV absorbers, such that the total fraction of UV absorbers is in the range from 0.1% to 40% by weight, more preferably in the range from 0.2% to 30% by weight and more preferably in the range 0.5% to 20% by weight, based on the total weight of the preparation. A (cosmetic and/or dermatological/pharmaceutical) preparation may comprise a total amount of UV filters and/or inorganic pigments such that the preparation has a sun protection factor of greater than or equal to 2 (preferably greater than or equal to 5) and further preferably up to 60. These sunscreens are suitable for protecting skin and hair.

Further suitable photoprotective agents (UV absorbers) are, for example, organic UV absorbers from the class of 4-aminobenzoic acid and derivatives, salicylic acid derivatives, benzophenone derivatives, dibenzoylmethane derivatives, diphenylacrylates, 3-imidazol-4-ylacrylic acid and its esters, benzofuran derivatives, benzylidenemalonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenyl, menthyl anthranilate, benzotriazole derivatives, indole derivatives.

Such preferred UV filters are as follows:
UVB filters such as, for example:
   - p-aminobenzoic acid
   - ethyl p-aminobenzoate (25 mol) ethoxylated
   - 2-ethylhexyl p-dimethylaminobenzoate
   - ethyl p-aminobenzoate (2 mol) N-propoxylated
   - glycerol p-aminobenzoate
   - homomenthyl salicylate (homosalate) (Neo Heliopan^{®}HMS)
   - 2-ethylhexyl salicylate (Neo Heliopan^{®}OS)
   - triethanolamine salicylate (Neo Heliopan® TS)
   - 4-isopropylbenzyl salicylate
   - menthyl anthranilate (Neo Heliopan^{®}MA)
   - ethyl diisopropylcinnamate
   - 2-ethylhexyl p-methoxycinnamate (Neo Heliopan^{®}AV)
   - methyl diisopropylcinnamate
   - isoamyl p-methoxycinnamate (Neo Heliopan^{®}E 1000)
   - p-methoxycinnamic acid diethanolamine salt
   - isopropyl p-methoxycinnamate
   - 3-(4'-trimethylammonium)benzylidenebornan-2-one methyl sulphate
   - ß-imidazole-4(5)-acrylic acid (urocanic acid)
   - 3-(4'-sulpho)benzylidenebornan-2-one and salts
   - 3-(4'-methylbenzylidene)-d,l-camphor (Neo Heliopan^{®}MBC)
   - 3-benzylidene-d,l-camphor
   - N-[(2 and 4)-[2-(oxoborn-3-ylidene)methyl]benzyl]acrylamide polymer
   - 4,4'-[(6-[4-(1,1-dimethyl)aminocarbonyl)phenylamino]-1,3,5-triazine-2,4-diyl)diimino]bis(benzoic acid 2-ethylhexyl ester) (Uvasorb^{®}HEB)
   - benzylidenemalonate-polysiloxane (Parsol^{®}SLX)
   - glyceryl ethylhexanoate dimethoxycinnamate
   - dipropylene glycol salicylate
   - tris(2-ethylhexyl) 4,4',4"-(1,3,5-triazine-2,4,6-triyltriimino)tribenzoate (Uvinul^{®}T150)
   - Benzylidene butyrolactones described in EP 1 008 593
   - Benzylidene-ß-dicarbonyl compounds described in WO 2005/107692
Broadband filters such as, for example:
   - 2-ethylhexyl 2-cyano-3,3-diphenylacrylate (Neo Heliopan®303)
   - ethyl 2-cyano-3,3'-diphenylacrylate
   - 2-hydroxy-4-methoxybenzophenone (Benzophenone-3, Oxybenzone) (Neo Heliopan® BB)
   - 2-hydroxy-4-methoxybenzophenone-5-sulfonic acid (sulisobenzone, Benzophenone-4) or its salts.
   - disodium 2,2'-dihydroxy-4,4'-dimethoxy-5,5'-disulphobenzophenone
   - phenol,-(2H-benzotriazol-2-yl-4-methyl-6-(2-methyl-3-(1,3,3,3-tetramethyl-1-(trimethylsilyl)oxy)disiloxyanyl)propyl), (Mexoryl^{®}XL)
   - 2,2'-methylenebis(6-(2H-benztriazol-2-yl)-4-1,1,3,3-tetramethylbutyl)-phenol), (Tinosorb^{®}M)
   - 2,4-bis[4-(2-ethylhexyloxy)-2-hydroxyphenyl]-1,3,5-triazine
   - 2,4-bis[{(4-(2-ethylhexyloxy)-2-hydroxy}phenyl]-6-(4-methoxyphenyl)-1,3,5-triazine, (Tinosorb^{®}S)
   - 2,4-bis[{(4-(3-sulphonato)-2-hydroxypropyloxy)-2-hydroxy}phenyl]-6-(4-methoxyphenyl)-1,3,5-triazine sodium salt
   - 2,4-bis[{(3-(2-propyloxy)-2-hydroxypropyloxy)-2-hydroxy}phenyl]-6-(4-methoxyphenyl)-1,3,5-triazine
   - 2,4-bis[{4-(2-ethylhexyloxy)-2-hydroxy}phenyl]-6-[4-(2-methoxyethyl-carbonyl)phenylamino]-1,3,5-triazine
   - 2,4-bis[{4-(3-(2-propyloxy)-2-hydroxypropyloxy)-2-hydroxy}phenyl]-6-[4-(2-ethylcarboxyl)phenylamino]-1,3,5-triazine
   - 2,4-bis[{4-(2-ethylhexyloxy)-2-hydroxy}phenyl]-6-(1-methylpyrrol-2-yl)-1,3,5-triazine
   - 2,4-bis[{4-tris(trimethylsiloxysilylpropyloxy)-2-hydroxy}phenyl]-6-(4-methoxyphenyl)-1,3,5-triazine
   - 2,4-bis[{4-(2"-methylpropenyloxy)-2-hydroxy}phenyl]-6-(4-methoxyphenyl)-1,3,5-triazine
   - 2,4-bis[{4-(1',1',1',3',5',5',5'-heptamethylsiloxy-2"-methylpropyloxy)-2-hydroxy}phenyl]-6-(4-methoxyphenyl)-1,3,5-triazine.
UVA filters such as, for example:
   - terephthalylidenedibornanesulphonic acid and salts (Mexoryl^{®}SX)
   - 4-t-butyl-4'-methoxydibenzoylmethane (avobenzone) (Neo Heliopan^{®}357)
   - hexyl 2-(4-diethylamino-2-hydroxybenzoyl)benzoate (Uvinul^{®} A Plus)
   - 4-isopropyldibenzoylmethane
   - menthyl anthranilate (Neo Heliopan^{®}MA)
   - indanylidene compounds as described in DE 100 55 940
   - Benzoylcinnamyl nitriles described in WO 2006/015954
UV absorbers particularly suitable for combination are as follows:
   - p-aminobenzoic acid
   - 3-(4'-trimethylammonium)benzylidenebornan-2-one methyl sulphate
   - homomenthyl salicylate (Neo Heliopan^{®}HMS)
   - terephthalylidenedibornanesulphonic acid and salts (Mexoryl^{®}SX)
   - 4-tert-butyl-4'-methoxydibenzoylmethane (Neo Heliopan^{®}357)
   - 3-(4'-sulpho)benzylidenebornan-2-one and salts
   - 2-ethylhexyl 2-cyano-3,3-diphenylacrylate (Neo Heliopan^{®}303)
   - N-[(2 and 4)-[2-(oxoborn-3-ylidene)methyl]benzyl]acrylamide polymer
   - 2-ethylhexyl p-methoxycinnamate (Neo Heliopan^{®}AV)
   - ethyl p-aminobenzoate (25 mol) ethoxylated
   - isoamyl p-methoxycinnamate (Neo Heliopan^{®}E1000)
   - 2,4,6-trianilino(p-carbo-2'-ethylhexyl-1'-oxy)-1,3,5-triazine (Uvinul^{®}T150)
   - phenol,2-(2H-benzotriazol-2-yl)-4-methyl-6-(2-methyl-3(1,3,3,3-tetramethyl-1-(trimethylsilyl)oxy)disiloxyanyl)propyl), (Mexoryl^{®}XL)
   - 4,4'-[(6-[4-(1,1-dimethyl)aminocarbonyl)phenylamino]-1,3,5-triazin-2,4-diyl)-diimino]bis(benzoic acid 2-ethylhexyl ester), (Uvasorb^{®} HEB)
   - 3-(4'-methylbenzylidene)-d,l-camphor (Neo Helipan^{®}MBC)
   - 3-benzylidenecamphor
   - 2-ethylhexyl salicylate (Neo Helipan^{®}OS)
   - 2-ethylhexyl 4-dimethylaminobenzoate (Padimate O)
   - 2-hydroxy-4-methoxybenzophenone (Benzophenone-3, Oxybenzone) (Neo Heliopan® BB
   - 2,2'-methylenebis(6-(2H-benzotriazol-2-yl)-4-1,1,3,3-tetramethylbutyl)-phenol), (Tinosorb^{®}M)
   - 2,4-bis[{(4-(2-ethylhexyloxy)-2-hydroxy}phenyl]-6-(4-methoxyphenyl)-1,3,5-triazine, (Tinosorb^{®}S)
   - benzylidenemalonate-polysiloxane (Parsol^{®}SLX)
   - menthyl anthranilate (Neo Heliopan^{®}MA)
   - hexyl 2-(4-diethylamino-2-hydroxybenzoyl)benzoate (Uvinul^{®} A Plus)
   - indanylidene compounds as described in DE 100 55 940
   - Benzoylcinnamyl nitriles described in WO 2006/015954
   - Benzylidene butyrolactones described in EP 1 008 593
   - Benzylidene-ß-dicarbonyl compounds described in WO 2005/107692

It is possible, furthermore, to use particulate UV filters or inorganic pigments, which if desired may have been rendered hydrophobic, such as the oxides of titanium (TiO₂), of zinc (ZnO), of iron (Fe₂O₃), of zirconium (ZrO₂), of silicon (SiO₂), of manganese (z.B. MnO), of aluminium (Al₂O₃), of cerium (e.g. Ce₂O₃) and/or mixtures.

The total amount of all sulfonated water soluble UV filters in the cosmetic or dermatological formulation, for example but not limited to, phenylbenzimidazole sulfonic acid, and/or Disodium Phenyl Dibenzimidazole Tetrasulphonic Acid and/or Benzophenone-4, and/or terephthalylidenedibornanesulphonic and/or 3-(4'-trimethylammonium)benzylidenebornan-2-one methyl sulphate, and/or 3-(4'-sulpho)benzylidenebornan-2-one, and their salts are in the range of 0.1 to 15.0% by weight and more particularly in the range from 0.5 to 10.0% and most particularly in the range of 1.0 to 8.0% by weight of the total formulation.

The amount of phenylbenzimidazole sulfonic acid and its salts used in the cosmetic or dermatological formulation containing troxerutin and disodium phenyl dibenzimidazole tetrasulfonate is in the range of 0.1 to 10.0% by weight, preferably in the range from 0.3 to 8 wt.-% and most preferably in the range from 0.5 to 5.0% of the total formulation.

The amount of Mexoryl^{®} SX and its salts used in the cosmetic or dermatological formulation containing troxerutin and disodium phenyl dibenzimidazole tetrasulfonate is in the range of 0.1 to 10.0wt.-%, preferably in the range from 0.3 to 8 wt.-% and most preferably in the range from 0.5 to 5.0 wt.-% of the total formulation.

The total amount of oil soluble UV filters that may be used in a cosmetic or dermatological formulation containing troxerutin and disodium phenyl dibenzimidazole tetrasulfonate, for example but not limited to (2-ethylhexyl) 4,4',4"-(1,3,5-triazine-2,4,6-triyltriimino)tribenzoate and /or -tert-butyl-4'-methoxydibenzoylmethane, and / or 2-ethylhexyl 4-dimethylaminobenzoate, and / or Mexoryl^{®}XL and/or Uvasorb^{®}HEB and/or Tinosorb^{®}S and/or Benzophenone-3 and/or Parsol^{®}SLX and/or Neo Heliopan^{®}MA, and /or isoamyl p-methoxycinnamate, and/or 2-ethylhexyl salicylate, and/or homosalate, and/or ethylhexyl methoxycinnamate, and/or octocrylene, and/or Uvinul^{®} A Plus, and/or 3-(4'-methylbenzylidene)-d,l-camphor, is in the range of 0.1 to 30 wt.-%, particularly in the range of 0.5 to 25 wt.-%, most particularly in the range of 1 to 20 wt.-% of the total formulation.

The amount of ethylhexyl methoxycinnamate used in the cosmetic or dermatological formulation containing troxerutin and disodium phenyl dibenzimidazole tetrasulfonate is in the range of 0.1 to 20.0 wt.%, preferably in the range from 0.3 to 15 wt.-% and most preferably in the range from 0.5 to 10.0 wt.-% of the total formulation.

The amount of isoamyl p-methoxycinnamate used in the cosmetic or dermatological formulation containing troxerutin and disodium phenyl dibenzimidazole tetrasulfonate is in the range of 0.1 to 20.0 wt.%, preferably in the range from 0.3 to 15 wt.-% and most preferably in the range from 0.5 to 10.0 wt.-% of the total formulation.

The amount of octocrylene used in the cosmetic or dermatological formulation containing troxerutin and disodium phenyl dibenzimidazole tetrasulfonate is in the range of 0.1 to 20.0 wt.-%, preferably in the range from 0.3 to 15 wt.-% and most preferably in the range from 0.5 to 10.0 wt.% of the total formulation.

The amount of salicylate esters used in the cosmetic or dermatological formulation containing troxerutin and disodium phenyl dibenzimidazole tetrasulfonate is in the range of 0.1 to 20.0 wt.-%, preferably in the range from 0.3 to 15 wt.-% and most preferably in the range from 0.5 to 10.0 wt.-% of the total formulation. When ethylhexyl salicylate is chosen as the UV filter, it is advantageous that its total amount ranges from 0.1 to 5.0 wt.-% of the formulation and when homosalate is chosen as the UV filter it is advantageous that its total amount ranges from 0.1 to 15.0 wt.-% of the formulation

The amount of butyl methoxydibenzoylmethane used in the cosmetic or dermatological formulation containing troxerutin and disodium phenyl dibenzimidazole tetrasulfonate is in the range of 0.1 to 10.0 wt.-%, preferably in the range from 0.3 to 7.0 wt.-% and most preferably in the range from 0.5 to 5.0 wt.-% of the total formulation.

The amount of Uvinul^{®} A Plus used in the cosmetic or dermatological formulation containing troxerutin and disodium phenyl dibenzimidazole tetrasulfonate is in the range of 0.1 to 10.0 wt.-%, preferably in the range from 0.3 to 7.0 wt.-% and most preferably in the range from 0.5 to 5.0 wt.-% of the total formulation.

The amount of Tinosorb^{®} S used in the cosmetic or dermatological formulation containing troxerutin and disodium phenyl dibenzimidazole tetrasulfonate is in the range of 0.1 to 10.0 wt.-%, preferably in the range from 0.3 to 7.0 wt.-% and most preferably in the range from 0.5 to 5.0 wt.-% of the total formulation.

The amount of Uvasorb^{®} HEB used in the cosmetic or dermatological formulation containing troxerutin and disodium phenyl dibenzimidazole tetrasulfonate is in the range of 0.1 to 10.0 wt.-%, preferably in the range from 0.3 to 7.0 wt.% and most preferably in the range from 0.5 to 5.0 wt.-% of the total formulation.

The amount of Uvinul^{®} T-150 used in the cosmetic or dermatological formulation containing troxerutin and disodium phenyl dibenzimidazole tetrasulfonate is in the range of 0.1 to 10.0 wt.-%, preferably in the range from 0.3 to 7.0 wt.-% and most preferably in the range from 0.5 to 5.0 wt.-% of the total formulation.

The total amount of oil microfine organic and/or inorganic pigments, for example but not limited to triazine derivatives and/or Zinc Oxide (coated and un-coated), and/or titanium dioxide (coated or un-coated) that may be used in a cosmetic or dermatological formulation containing troxerutin and disodium phenyl dibenzimidazole tetrasulfonate is in the range of 0.1 to 20.0 wt.-%, preferably in the range from 0.3 to 15 wt.-% and more preferably in the range from 0.5 to 10.0 wt.-% and most preferably in the range from 0.75 wt.-% to 7.5 wt.-%. When titanium dioxide is chosen as the UV filter, it is advantageous that its total amount ranges from 0.1 to 10.0 wt.-% of the formulation. When zinc oxide is chosen as the UV filter it is advantageous that its total amount ranges from 0.1 to 10.0 wt.-% of the formulation and when one or more triazine organic pigment(s) are chosen it is advantageous that its total amount ranges from 0.1 to 10.0 wt.-% of the formulation.

Combining troxerutin and disodium phenyl dibenzimidazole tetrasulfonic acid or one or more of its salts with other UV filters, for example with the UV filters listed above and particularly with the UV filters listed as "particularly suitable for combination", but not limited to these, leads to synergistic effects in the degree of protection offered against UVB and UVA radiation as determined by measurements to determine sun protection factors against UVA and / or UVB radiation. This disclosure thus also provides the teaching that combining (a) troxerutin and (b) disodium phenyl dibenzimidazole tetrasulfonic acid or any of its salts with individual or any desired mixtures of any of the UV filters listed above as well as any from the allowed UV filters for use in sun protection products legislated in :

| | |
|---|---|
| USA: | by the Food and Drug Administration (FDA).published in the Monograph for Sunscreen Drug Products for Over-The-Counter Human Use. |
| Europe: | by the Cosmetics Directive 76/768 EEC of the Council of European Communities published in the in the Official Journal of the European Communities. |
| Japan: | in the positive list of allowed UV filters in the publication of the cosmetic criteria by the Ministry of Health and Welfare (MHW). |
| Australia: | in the positive list of allowed UV filters published by the Australian Therapeutic Goods Administration in the Australian Register of Therapeutic Goods (ARTG). |

will lead to synergistic protective effects against UVA and/or UVB radiation.

Particularly in cosmetic and dermatological preparations, troxerutin absorbs UV radiation, when it is used in combination with disodium phenyl dibenzimidazole tetrasulfonate it also leads to photostabilisation of UV absorbers of lower stability to UV such as derivatives of butyl methoxydibenzoylmethane. In particular, troxerutin and its salts together with disodium phenyl dibenzimidazole tetrasulfonate or its acid form give rise to stabilisation of the UVA absorber 4-tert.-butyl-4'-methoxydibenzoylmethane, which is highly unstable to light.

It is furthermore advantageous to add to this three-compound combination one or more highly photostable UV absorbers, such as, for example, methylbenzylidene-camphor, 2-ethylhexyl-2-cyano-3,3'-diphenylacrylate, octyltriazone, Uvasorb^{®}HEB, Tinosorb^{®}S, Tinosorb^{®}M, ethylhexyl salicylate, homomenthyl salicylate, phenylenbenzimidazolesulphonic acid, Benzophenone-4, Uvinul A Plus, Mexoryl^{®}SX, Mexoryl^{®}XL, Parsol^{®}SLX or indanylidene compounds as described in DE 100 55 940 and/or WO 02/38537, or benzoylcinnamyl nitriles as described in WO 2006/015954, or benzylidene butyrolactones as described in EP 1 008 593, or benzylidene-ß-dicarbonyl compounds as described in WO 2005/107692.

It is furthermore advantageous to add, alone or in addition to the UV filters listed above, the photostabilising emollient 2,6-diethylhexyl naphthalate sold under the trade name of Corapan^{®} TQ by Symrise to improve the photostability of butyl methoxydibenzoylmethane.

The combination of troxerutin and disodium phenyl dibenzimidazole tetrasulfonic acid and/or any of its salts together with UVA absorbers, especially UVA-I absorbers, provides comprehensive protection against UVA radiation (320-400 nm). A combination of troxerutin and disodium phenyl dibenzimidazole tetrasulfonic acid and/or any of its salts together with butyl methoxydibenzoylmethane, or Tinosorb S or indanylidene derivatives or benzoylcinnamyl nitriles (UVA-I absorbers) is to be mentioned in particular for broad UV protection. Further UVA filters that are preferred to be used in combination with both troxerutin and disodium phenyl dibenzimidazole tetrasulfonic acid and/or any of its salts, on their own or in combination, are Mexoryl SX, Mexoryl^{®}XL, Tinosorb^{®}M, Tinosorb^{®}S, Benzophenone-3, Benzophenone-4, Uvinul^{®} A Plus, Neo Heliopan^{®}357 and Neo Heliopan^{®}MA.

The abovementioned combinations can be combined with all UVB filters and mixtures of these filters (cf. the above mentioned particularly suitable UV absorbers) for optimum broadband protection against UVA and UVB radiation. Neo Heliopan^{®}AV, Neo Heliopan^{®}E1000, Neo Heliopan^{®}Hydro, Neo Heliopan^{®}MBC, Neo Heliopan^{®}303, Neo Heliopan^{®}OS, Neo Heliopan^{®}HMS, Uvinul^{®}T150, Uvasorb^{®}HEB, Parsol^{®} SLX are particularly suitable UVB filters.

Synergies of both troxerutin and disodium phenyl dibenzimidazole tetrasulfonate together with other constituents that do not absorb UV light, with regard to an improved protection against UV light, are to be expected.

Cosmetic and dermatological preparations in the sense of this disclosure contain one or more conventional UVA, UVB and/or broadband filters as single substances or in arbitrary mixtures with one another in the lipid phase and/or in the aqueous phase. They are satisfactory products in every respect which, surprisingly are distinguished by high UVA protection and a high UVB protection factor.

It is highly disadvantageous if UV absorbers leave marks on articles of clothing that can no longer be washed out. In particular, the UVA absorber tert.-butyl-methoxydibenzoylmethane is known to produce marks on textiles that can no longer be washed out. The use of a of troxerutin and disodium phenyl dibenzimidazole tetrasulfonate according to the invention do not have this disadvantage since any marks formed on textiles can be washed out very readily. (Cosmetic and/or pharmaceutical/dermatological) preparations can be formulated in the customary manner and serve as cosmetic and/or dermatological sunscreens and also for the treatment, care and cleansing of the skin and/or the hair and as a make-up product in decorative cosmetics.

These cosmetic and pharmaceutical preparations serving for protection of skin and hair against UV radiation can be in the use forms conventionally used, i.e. in the form of oil-in-water, water-in-oil or mixed emulsion, in the form of milk, in the form of lotion or cream, aerosol, hydrodispersion gel or oil gel (emulsifier-free), spray, foam, solution, powder, pencil preparation or in the form of any other customary cosmetic or pharmaceutical formulation. Preparations such as shampoo, rinse, conditioner, gel, lotion, spray or cream are preferably used for protection of the hair against UV rays. (Cosmetic and/or pharmaceutical/dermatological) preparations can have the customary composition and can be used for cosmetic and/or dermatological sun protection, and also for the treatment, care and cleansing of the skin and/or of the hair and as a make-up product in decorative cosmetics. Accordingly, the preparations can, depending on their formulation, be used, for example, as skin protection cream, cleansing milk, sunscreen lotion, nourishing cream, day cream or night cream. The preparations can, depending on their formulation, also be used for example, in hair care compositions such as shampoos, conditioners, 2 in 1 preparations, anti-dandruff shampoos, hair tonics, hair lotions, hair rinses, styling products, sprays. In some instances, it is possible and advantageous to use the preparations as bases for pharmaceutical preparations. Preference is given, in particular, to those cosmetic and dermatological preparations in the form of a skin care, hair care or make-up product. Typical embodiments are creams, gels e.g. but not limited to hydrogels, hydrodispersion gels, oil gels; lotions, alcoholic and aqueous/alcoholic solutions, emulsions in their various forms for example but not limited to oil in water (O/W), water in oil (W/O), mixed emulsions, PIT emulsions, Pickering emulsions, microemulsions, nano-emulsions; aerosol foams, non-aerosol foams, aerosols sprays, non-aerosol sprays, pump sprays, serums, rollons, pastes, balsams, or stick preparations. These compositions may also comprise, as further auxiliaries and additives, mild surfactants, co-emulsifiers, super-fatting agents, pearlescent waxes, bodying agents, thickeners, polymers, silicone compounds, fats, waxes, stabilizers, biogenic active ingredients, deodorant active ingredients, antidandruff agents, film formers, swelling agents, hydrotropic agents, preservatives, insect repellents, tanning agents, artificial self-tanning agents (e.g. dihydroxyacetone), stabilizers, perfume oils, dyes, antimicrobial agents, aqueous and non-aqueous plant extracts. For use, the cosmetic and dermatological preparations are applied to the skin and/or the hair in a sufficient amount in the manner customary for cosmetics or pharmacological and dermatological preparations.

More preference is given to those cosmetic and dermatological preparations in the form of a cosmetic composition for the protection of the skin and hair. Advantageously, in addition to troxerutin and disodium phenyl dibenzimidazole tetrasulfonate, these can contain at least one inorganic pigment, preferably an inorganic micropigment. Those cosmetic and dermatological preparations that are in the form of a skin care or make-up product are particularly preferred.

The cosmetic and dermatological preparations can comprise cosmetic auxiliaries, as are customarily used in such preparations, e.g. preservatives, bactericides, perfumes, antifoams, dyes, pigments which have a coloring action, thickeners, moisturizers and/or humectants, fats, oils, waxes or other customary constituents of a cosmetic or dermatological formulation, such as alcohols, polyols, polymers, foam stabilizers, electrolytes, organic solvents or silicone derivatives. The amounts of cosmetic or dermatological auxiliaries and carrier substances and perfume which can be used in each case can be determined easily by the person skilled in the art by simple trial and error, depending on the nature of the product in question.

Preferred cosmetic and/or pharmaceutical, especially dermatological preparations may also comprise anionic, cationic, nonionic and/or amphoteric surfactants. Surfactants are amphiphilic substances which can dissolve organic, nonpolar substances in water. In this context, the hydrophilic components of a surfactant molecule are usually polar functional groups, for example -COO⁻, -OSO₃²⁻, -SO₃⁻, while the hydrophobic parts as a rule are nonpolar hydrocarbon radicals. Surfactants are in general classified according to the nature and charge of the hydrophilic molecular moiety. A distinction can be made between four groups here:
- anionic surfactants,
- cationic surfactants,
- amphoteric surfactants and
- nonionic surfactants.

Anionic surfactants as a rule contain carboxylate, sulphate or sulphonate groups as functional groups. In aqueous solution, they form negatively charged organic ions in an acid or neutral medium. Cationic surfactants are almost exclusively characterized by the presence of a quaternary ammonium group. In aqueous solution, they form positively charged organic ions in an acid or neutral medium. Amphoteric surfactants contain both anionic and cationic groups and accordingly behave like anionic or cationic surfactants in aqueous solution, depending on the pH. In a strongly acid medium they have a positive charge, and in an alkaline medium a negative charge. On the other hand, they are zwitterionic in the neutral pH range. Polyether chains are typical of nonionic surfactants. Nonionic surfactants do not form ions in an aqueous medium.

### A. Anionic surfactants

Anionic surfactants which are advantageously used are acylamino acids (and salts thereof), such as:
   - acyl glutamates, for example sodium acyl glutamate, di-TEA-palmitoyl aspartate and sodium caprylic/capric glutamate,
   - acyl peptides, for example palmitoyl hydrolysed milk protein, sodium cocoyl hydrolysed soya protein and sodium/potassium cocoyl hydrolysed collagen,
   - sarcosinates, for example myristoyl sarcosine, TEA-lauroyl sarcosinate, sodium lauroyl sarcosinate and sodium cocoyl sarcosinate,
   - taurates, for example sodium lauroyl taurate and sodium methylcocoyl taurate,
   - acyl lactylates, lauroyl lactylate, caproyl lactylate
   - alaninates
carboxylic acids and derivatives, such as for example:
   - TEA stearate, Glyceryl stearates, PEG glyceryl stearates
      lauric acid, aluminium stearate, magnesium alkanolate and zinc undecylenate,
      ester-carboxylic acids, for example calcium stearoyl lactylate, laureth-6 citrate and sodium PEG-4 lauramide carboxylate, glyceryl stearates, glyceryloleylstearates, glyceryl citrates, glyceryl oleyl citrates,
   - ether-carboxylic acids, for example sodium laureth-13 carboxylate and sodium PEG-6 cocamide carboxylate,
Glucoside esters, such as for example
   - cetearyl glucoside, lauryl glucoside
phosphoric acid esters and salts, such as, for example :
   - cetyl phosphate (mono, di cetyl and their mixtures), Potassium cetyl phosphate, (mono, di cetyl and their mixtures), DEA cetyl phosphate (mono, di cetyl and their mixtures), DEA -oleth-10 phosphate and dilaureth-4 phosphate,
sulphonic acids and salts, such as
   - acyl isethionates, e.g. sodium/ammonium cocoyl isethionate,
   - alkylarylsulphonates,
   - alkylsulphonates, for example sodium coco-monoglyceride sulphate, sodium C12-14 olefinsulphonate, sodium lauryl sulphoacetate and magnesium PEG-3 cocamide sulphate,
   - sulphosuccinates, for example dioctyl sodium sulphosuccinate, disodium laureth-sulphosuccinate, disodium laurylsulphosuccinate and disodium undecylenamido-MEA-sulphosuccinate
      and
sulphuric acid esters, such as:
   - alkyl ether sulphate, for example sodium, ammonium, magnesium, MIPA, TIPA laureth sulphate, sodium myreth sulphate and sodium C12-13 pareth sulphate,
   - alkyl sulphates, for example sodium, ammonium and TEA lauryl sulphate.

### B. Cationic surfactants

Cationic surfactants which are advantageously used are
- alkylamines,
- alkylimidazoles,
- ethoxylated amines,
- quaternary surfactants,
- RNH₂CH₂CH₂COO⁻ (at pH=7)
- RNHCH₂CH₂COO- B⁺ (at pH=12) B⁺ = any desired cation, e.g. Na⁺ and
- ester quats.

Quaternary surfactants contain at least one N atom which is covalently bonded to 4 alkyl or aryl groups. This leads to a positive charge, independently of the pH. Alkylbetaine, alkylamidopropylbetaine and alkylamidopropylhydroxysulphaine are advantageous. The cationic surfactants used can further preferably be chosen from the group consisting of quaternary ammonium compounds, in particular benzyltrialkylammonium chlorides or bromides, such as, for example, benzyldimethylstearylammonium chloride, and also alkyltrialkylammonium salts, for example cetyltrimethylammonium chloride or bromide, alkyldimethylhydroxyethylammonium chlorides or bromides, dialkyldimethylammonium chlorides or bromides, alkylamideethyltrimethylammonium ether sulphates, alkylpyridinium salts, for example lauryl- or cetylpyridinium chloride, imidazoline derivatives and compounds having a cationic character, such as amine oxides, for example alkyldimethylamine oxides or alkylaminoethyldimethylamine oxides. Cetyltrimethyl-ammonium salts in particular are advantageously used.

### C. Amphoteric surfactants

Amphoteric surfactants which are advantageously to be used are
- a.cyl/dialkylethylenediamine, for example sodium acylamphoacetate, disodium acylamphodipropionate, disodium alkylamphodiacetate, sodium acylamphohydroxypropylsulphonate, disodium acylamphodiacetate and sodium acylamphopropionate,
- N-alkylamino acids, for example aminopropyl alkylglutamide, alkylaminopropionic acid, sodium alkylimidodipropionate and lauroamphocarboxyglycinate.
- acylamphohydroxypropylsulphonate, disodium acylamphodiacetate and sodium acylamphopropionate,
- N-alkylamino acids, for example aminopropyl alkylglutamide, alkylaminopropionic acid, sodium alkylimidodipropionate and lauroamphocarboxyglycinate.

### D. Nonionic surfactants

Nonionic surfactants which are advantageously used are
- alcohols,
- alkanolamides, such as cocamides MEA/DEA/MIPA,
- amine oxides, such as cocoamidopropylamine oxide,
- ethers, for example ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated/propoxylated lanolin, ethoxylated/propoxylated polysiloxanes, propoxylated POE ethers and alkyl polyglycosides, such as lauryl glucoside, decyl glucoside and coco-glycoside.
- sucrose esters, sucrose ethers
- polyglycerol esters, diglycerol esters, monoglycerol esters polyglyceryl-2 dipolyhydroxystearate (Dehymuls^{®}PGPH), polyglyceryl-3 diisostearate (Lameform^{®}TGI), polyglyceryl-4 isostearate (Isolan^{®}GI 34), polyglyceryl-3 oleate, diisostearyl polyglyceryl-3 diisostearate (Isolan^{®}PDI), polyglyceryl-3 methylglucose distearate (Tego Carey^{®}450), polyglyceryl-3 beeswax (Cera Bellina^{®}), polyglyceryl-4 caprate (polyglycerol caprate T2010/90), polyglyceryl-3 cetyl ether (Chimexane^{®}NL), polyglyceryl-3 distearate (Cremophor^{®}GS 32), polyglyceryl-2 stearate (Hostacerin^{®}DGMS) and polyglyceryl polyricineoleate (Admul^{®}WOL 1403), and mixtures thereof.
- methylglucose esters, esters of hydroxy acids

The use of a combination of anionic and/or amphoteric surfactants with one or more nonionic surfactants is further advantageous.

In addition, cosmetic and dermatological preparations may advantageously, but not obligatorily, comprise inorganic pigments based on finely disperse metal oxides and/or other metal compounds which are insoluble or sparingly soluble in water, in particular the oxides of titanium (TiO₂), zinc (ZnO), iron (e.g. Fe₂O₃), zirconium (ZrO₂), silicon (SiO₂), manganese (e.g. MnO), aluminum (Al₂O₃), cerium (e.g. Ce₂O₃), mixed oxides of the corresponding metals, and mixtures of such oxides. These pigments are X-ray-amorphous or non-X-ray-amorphous. More preference is given to pigments based on TiO₂. X-ray-amorphous oxide pigments are metal oxides or semi-metal oxides which reveal no or no recognizable crystalline structure in X-ray diffraction experiments. Such pigments are often obtainable by flame reaction, for example by reacting a metal or semi-metal halide with hydrogen and air (or pure oxygen) in a flame.

In cosmetic, dermatological or pharmaceutical preparations, X-ray-amorphous oxide pigments are used as thickeners and thixotropic agents, flow auxiliaries for emulsion and dispersion stabilization and as carrier substance (for example for increasing the volume of finely divided powders). X-ray-amorphous oxide pigments which are known and often used in cosmetic or dermatological galenics are, for example, high-purity silicon oxide. Preference is given to high-purity, X-ray-amorphous silicon dioxide pigments with a particle size in the range from 5 to 40 nm and an active surface area (BET) in the range from 50 to 400 m²/g, preferably 150 to 300 m²/g, where the particles are to be regarded as spherical particles of very uniform dimension. Macroscopically, the silicon dioxide pigments are recognizable as loose, white powders. Silicon dioxide pigments are sold commercially under the name Aerosil^{®} (CAS-No. 7631-85-9) or Carb-O-Sil Advantageous Aerosil^{®} grades are, for example, Aerosil^{®}0X50, Aerosil^{®}130, Aerosil^{®}150, Aerosil^{®}200, Aerosil^{®}300, Aerosil^{®}380, Aerosil^{®}MQX 80, Aerosil^{®} MOX 170, Aerosil^{®}COK 84, Aerosil^{®} R 202, Aerosil^{®}R 805, Aerosil^{®}R 812, Aerosil^{®}R 972, Aerosil^{®}R 974, Aerosil^{®}R976. Cosmetic or dermatological light protection preparations may comprise 0.1 to 20% by weight, advantageously 0.5 to 10% by weight, more preferably 1 to 5% by weight, of X-ray-amorphous oxide pigments. The non-X-ray-amorphous inorganic pigments are advantageously in hydrophobic form, i.e. have been surface-treated to repel water. This surface treatment may involve providing the pigments with a thin hydrophobic layer by processes known per se. Such a process involves, for example, producing the hydrophobic surface layer by a reaction according to

n TiO₂ + m (RO)₃Si-R' → n TiO₂ (surf.)

where n and m are stoichiometric parameters to be used as desired, and R and R' are the desired organic radicals. Hydrophobicized pigments prepared analogously to DE-A 33 14 742, for example, are advantageous.

For example, mention may be made of TiO₂ pigments, as are sold under the trade name T805 from Degussa. Preference is also given to TiO₂/Fe₂O₃ mixed oxides, as are supplied, for example, under the trade name T817, also from Degussa.

The total amount of inorganic pigments, in particular hydrophobic inorganic micropigments, in the finished cosmetic or dermatological preparations is advantageously chosen from the range from 0.1 to 30% by weight, preferably 0.1 to 10.0% by weight, preferably 0.5 to 6.0% by weight, based on the total weight of the preparations.

An additional content of skin lightening ingredients in the cosmetic or dermatological preparation is optional. Such skin lightening ingredients which can be used are for example but not limited to the following : kojic acid (5-hydroxy-2-hydroxymethyl-4-pyranone), kojic acid derivatives such as for example kojic dipalmitate, arbutin, ascorbic acid, ascorbic acid derivatives, hydroquinone, hydroquinone derivatives, styryl resorcinol derivatives (e.g. 4-(1-phenylethyl)1,3-benzenediol), molecules containing sulphur, such as glutathione or cysteine for example, alpha-hydroxy acids (e.g. citric acid, lactic acid, malic acid) and their derivatives, N-acetyltyrosine and derivatives, undecenoylphenylalanine, gluconic acid, chromone derivatives such as aloesin, flavonoids, thymol derivatives, 1-aminoethylphosphinic acid, thiourea derivatives, ellagic acid, nicotinamide, zinc salts such as zinc chloride or zinc gluconate for example, thujaplicin and derivatives, triterpenes such as maslic acid, sterols such as ergosterol, benzofuranones such as senkyunolide, vinyl- and ethylguaiacol, dionic acids such as octodecenedionic acid and azelaic acid, nitrogen oxide synthesis inhibitors such as L-nitroarginine and its derivatives, 2,7-dinitroindazole or thiocitrulline, metal chelators (e.g. alpha-hydroxy fatty acids, palmitic acid, phytic acid, lactoferrin, humic acid, gallic acid, bile extracts, bilirubin, biliverdin), retinoids, soja milk, soya extract, serine protease inhibitors or lipoic acid or other synthetic or natural active compounds for skin and hair lightening, these compounds also being used in the form of an extract from plants, such as bearberry extract, rice extract, papaya extract, liquorice root extract or constituents concentrated from these, such as glabridin or licochalcone A, Artocarpus extract, extract from Rumex and Ramulus species, extracts from pine species (Pinus) and extracts from Vitis species or stilbene derivatives concentrated from these, extract from saxifraga, mulberry, Scutelleria and/or grapes.

An additional content of antioxidants in the cosmetic or dermatological preparation is generally preferred. Favorable antioxidants which can be used are all antioxidants customary or suitable for cosmetic and/or dermatological applications.

The antioxidants are advantageously chosen from the group of amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximines, homocysteine sulfoximine, buthionine sulfones, penta-, hexa-, heptathionine sulfoximine) in very low tolerated doses (e.g. pmol to µmol/kg), and also (metal) chelating agents (e.g. α-hydroxy fatty acids, palmitic acid, phytic acid, lactoferrin), α-hydroxy acids (e.g. citric acid, lactic acid, maleic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate), and coniferyl benzoate of benzoin resin, rutinic acid and derivatives thereof, α-glycosylrutin, ferulic acid, furfurylideneglucitol, carnosine, butylhydroxy-toluene, butylhydroxyanisol, nordihydroguaiacic acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids) of the active ingredients. The amount of the above-mentioned antioxidants (one or more compounds) in the preparations is preferably 0.001 to 30% by weight, more preferably 0.05 to 20% by weight, and most preferably 1 to 10% by weight, based on the total weight of the preparation.

Preferred cosmetic and/or pharmaceutical, especially dermatologically active preparations may advantageously also comprise vitamins and vitamin precursors, it being possible for all the vitamins and vitamin precursors which are suitable or usual for cosmetic and/or dermatological applications to be used. Those worth mentioning here are, in particular, vitamins and vitamin precursors, such as tocopherols, vitamin A, niacin acid and niacinamide, further vitamins of the B complex, in particular biotin, and vitamin C and panthenol and derivatives thereof, in particular the esters and ethers of panthenol, and cationically derivatized panthenols, such as panthenol triacetate, panthenol monoethyl ether and the monoacetate thereof and cationic panthenol derivatives. If vitamin E and/or derivatives thereof represent the antioxidant(s), it is advantageous to choose their respective concentrations from the range from 0.001 to 10% by weight, based on the total weight of the formulation. If vitamin A or vitamin A derivatives, or carotenes or derivatives thereof represent the antioxidant(s), it is advantageous to choose their respective concentrations from the range from 0.001 to 10% by weight, based on the total weight of the formulation.

Preferred cosmetic and/or pharmaceutical, especially dermatological preparations may also comprise lipids chosen from the following group of substances:
(i) linear or branched saturated paraffins (mineral oils) having 15 or more C atoms, in particular having 18 to 45 C atoms;
(ii) esters having 12 or more C atoms of linear or branched fatty acids having 6 to 30 C atoms and linear or branched, saturated or unsaturated mono-, di-or triols having 3 to 30 C atoms, these esters having no free hydroxyl groups;
(iii) esters of benzoic acid and linear or branched, saturated or unsaturated monoalkanols having 8 to 20 C atoms;
(iv) monoesters or diesters of alcohols having 3 to 30 C atoms and naphthalene-monocarboxylic or -dicarboxylic acids; especially naphthalenemonocarboxylic acid C₆-C₁₈ esters and naphthalenedicarboxylic acid di-C₆-C₁₈ esters;
(v) linear or branched, saturated or unsaturated di-C₆-C₁₈-alkyl ethers;
(vi) silicone oils;
(vii) 2-alkyl-1-alkanols of the formula (III) 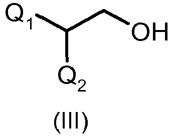 where
   Q₁ is a linear or branched alkyl radical having 6 to 24 C atoms and
   Q₂ is a linear or branched alkyl radical having 4 to 16 C atoms.

An oil phase or oil component in the narrower (and preferred) sense encompasses the following groups of substances:
(i) linear or branched, saturated paraffins having 20 to 32 C atoms;
(ii) esters having at least 14 C atoms of linear or branched, saturated fatty acids having 8 to 24 C atoms and linear or branched, saturated or unsaturated mono-, di- or triols having 3 to 24 C atoms, these esters containing no free hydroxyl groups;
(iii) esters of benzoic acid and linear or branched, saturated monoalkanols having 10 to 18 C atoms;
(iv) 2,6-naphthalenedicarboxylic acid di-C6-C12 esters;
(v) linear or branched, saturated di-C6-C18-alkyl ethers, especially (straight-chain) di-C6-C12-alkyl ethers;
(vi) silicone oils from the group of the cyclotrisiloxanes, cyclopentasiloxanes, dimethylpolysiloxanes, diethylpolysiloxanes, methylphenylpolysiloxanes, diphenylpolysiloxanes and hybrid forms thereof;
(vii) 2-alkyl-1-alkanols having 12 to 32 C atoms of the formula (III)
   where
   Q₁ is a (preferably linear) alkyl radical having 6 to 18 C atoms and
   Q₂ is a (preferably linear) alkyl radical having 4 to 16 C atoms.

An oil phase in the narrowest (and most preferred) sense encompasses the following groups of substances:
(i) linear or branched, saturated paraffins having 20 to 32 C atoms such as isoeicosane or squalane;
(ii) esters having at least 16 C atoms of linear or branched, saturated fatty acids having 8 to 18 C atoms and linear or branched, saturated mono-, di- or triols having 3 to 18 C atoms, these esters containing no free hydroxyl groups;
(iii) esters of benzoic acid and linear or branched, saturated monoalkanols having 12 to 15 C atoms, especially C₁₂-₁₅-alkyl benzoates;
(iv) 2,6-naphthalenedicarboxylic acid di-C6-C10 esters, especially diethylhexyl 2,6-naphthalenedicarboxylate;
(v) straight-chain di-C₆-C₁₀-alkyl ethers; especially di-n-octyl ether (dicaprylyl ether);
(vi) silicone oils from the group undecamethylcyclotrisiloxane, cyclomethicone, decamethylcyclopentasiloxane, dimethylpolysiloxanes, diethylpolysiloxanes, methylphenylpolysiloxanes and diphenylpolysiloxanes;
(vii) 2-alkyl-1-alkanols having 12 to 32 C atoms of the formula (III)
   where
   Q₁ is a (preferably linear) alkyl radical having 6 to 18 C atoms and
   Q₂ is a (preferably linear) alkyl radical having 4 to 16 C atoms.

Particularly preferred components of type (i) in the oil phase are as follows: isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate, 2-ethylhexyl isostearate, isotridecyl isononanoate, 2-ethylhexyl cocoate, caprylic/capric triglyceride, and also synthetic, semisynthetic and natural mixtures of such esters, e.g. jojoba oil.

Fatty acid triglycerides (oil components of type (i) in the oil phase) may also be in the form of, or in the form of a constituent of, synthetic, semisynthetic and/or natural oils, examples being olive oil, sunflower oil, soya oil, peanut oil, rapeseed oil, almond oil, palm oil, coconut oil, palm kernel oil and mixtures thereof.

Particularly preferred oil components of type (vii) in the oil phase are as follows: 2-butyl-1-octanol, 2-hexyl-1-decanol, 2-octyl-1-dodecanol, 2-decyltetradecanol, 2-dodecyl-1-hexadecanol and 2-tetradecyl-1-octadecanol.

Particularly preferred oil components in the oil phase are mixtures comprising C₁₂-C₁₅-alkyl benzoate and 2-ethylhexyl isostearate, mixtures comprising C₁₂-C₁₅-alkyl benzoate and isotridecyl isononanoate, mixtures comprising C₁₂-C₁₅-alkyl benzoate, 2-ethylhexyl isostearate and isotridecyl isononanoate, mixtures comprising cyclomethicone and isotridecyl isononanoate, and mixtures comprising cyclomethicone and 2-ethylhexyl isostearate.

Preferred cosmetic and/or pharmaceutical, especially dermatologically active preparations may advantageously also comprise the use of polymers to imrove the spreadibility of the formulation upon the skin or hair, or improve the water and or sweat and or rub-off resistancy of the formula and to improve the protection factor of the formulation. Examples of such polymers are : VP/Eicosene copolymers sold under the trade name of Antaron V-220 by International Speciality Products, VP/Hexadecene copolymer sold under the trade names Antaron V-216 and Antaron V-516 by International Speciality Products, Tricontanyl PVP sold under the trade name of Antaron WP-660 by International Speciality Products, Isohexadecane and Ethylene/Propylene/Styrene copolymer and Butylene/Styrene copolymer sold under the trade names of Versagel MC and MD by Penreco, Hydrogenated polyisobutene and and Ethylene/Propylene/Styrene copolymer and Butylene/Styrene co-polymer sold under the trade mane of Versagel ME by Penreco, Acrylates/Octylacrylamide Coploymers sold under the trade name of Dermacryl 79, Dermacryl AQF and Dermacryl LT by National Starch, Polyurethanes such as PPG-17/IPDI/DMPA copolymer sold under the trade name of Avalure UR 450 & 525 sold by Noveon, Polyurethanes-2 and -4 sold under the trade names Avalure UR-405, -410, - 425, -430 and - 445 525 sold by Noveon, Polyurethane 5 and Butyl Acetate and isopropyl alcohol sold under the trade name Avalure UR - 510 and - 525 sold by Noveon, Polyurethanes -1 and - 6 sold under the trade name of Luviset PUR by BASF, Hydrogenated Dimer Dilinoleyl/Dimethylcarbonate Copolymer sold under the trade name of Cosmedia DC by Cognis.

Of course, as one well versed in the art of cosmetic and dermatological formulation knows, this is not an exhaustive list and other suitable polymers not listed here may be used. Examples of such polymers may be found in the latest edition of the CTFA's International Cosmetic Ingredient Dictionary

The amount of polymers used to obtain the desired effect in the formulation range from 0.10% to 5.0% by weight of the fomulation and especially in the range from 0.25% to 3.0% by weight of the formulation.

Preferred cosmetic and/or pharmaceutical, especially dermatologically active preparations comprise, if desired, further ingredients having care properties, such as, for example, fatty alcohols having 6 to 30 C atoms. The fatty alcohols here can be saturated or unsaturated and linear or branched. Furthermore, these fatty alcohols can in some cases be part of the oil phase (III) if they correspond to the definition given there. Alcohols which can be employed are, for example, decanol, decenol, octanol, octenol, dodecanol, dodecenol, octadienol, decadienol, dodecadienol, oleyl alcohol, ricinoleyl alcohol, erucyl alcohol, stearyl alcohol, isostearyl alcohol, cetyl alcohol, lauryl alcohol, myristyl alcohol, arachidyl alcohol, caprylyl alcohol, capryl alcohol, linoleyl alcohol, linolenyl alcohol and behenyl alcohol, and also Guerbet alcohols thereof, such as, for example, 2-octyl-1-dodecanol, it being possible for the list to be extended virtually as desired by further alcohols of related structural chemistry. The fatty alcohols preferably originate from natural fatty acids, being conventionally prepared from the corresponding esters of the fatty acids by reduction. Fatty alcohol fractions which are formed by reduction from naturally occurring fats and fatty oils, such as beef tallow, peanut oil, colza oil, cottonseed oil, soya oil, sunflower oil, palm kernel oil, linseed oil, maize oil, castor oil, rapeseed oil, sesame oil, cacao butter and coconut fat, can further be employed.

Substances having care properties which advantageously can be employed in the cosmetic and/or dermatologically active preparations can further include
- ceramides, where ceramides are understood as meaning N-acylsphingosins (fatty acid amides of sphingosin) or synthetic analogues of such lipids (so-called pseudo-ceramides), which significantly improve the water retention capacity of the stratum corneum.
- phospholipids, for example soya lecithin, egg lecithin and cephalins
- fatty acids
- phytosterols and phytosterol-containing fats or waxes
- vaseline, paraffin oils and silicone oils; the latter include, inter alia, dialkyl- and alkylarylsiloxanes, such as dimethylpolysiloxane and methylphenyl-polysiloxane, and also alkoxylated and quaternised derivatives thereof.

Animal and/or plant protein hydrolysates can advantageously also be added to preferred cosmetic and/or pharmaceutical, especially dermatologically active, preparations. Substances which are advantageous in this respect are, in particular, elastin, collagen, keratin, milk protein, soya protein, oat protein, pea protein, almond protein and wheat protein fractions or corresponding protein hydrolysates, and also condensation products thereof with fatty acids, and quaternised protein hydrolysates, the use of plant protein hydrolysates being preferred.

The aqueous phase of the preparations optionally advantageously comprises alcohols, diols or polyols (lower alkyl), and ethers thereof, preferably ethanol, isopropanol, propylene glycol, 1,2-hexanediol, 1,2-octanediol, 1,2-decanediol, a mixture of 1,2-hexanediol and 1,2-octanediol, a mixture of 1,2-hexanediol and 1,2-decanediol, a mixture of 1,2-octanediol and 1,2-decanediol, a mixture of 1,2-hexanediol, 1,2-octanediol and 1,2-decanediol, glycerol, ethylene glycol-monoethyl or monobutyl ether, propylene glycol mono-methyl, -monoethyl or monobutyl ether, diethylene glycol monomethyl or - monoethyl ether and analogous products, and also alcohols (lower alkyl), e.g. ethanol, 1,2-propanediol, glycerol, and, in particular, one or more thickeners which can advantageously be chosen from the group of silicon dioxide, aluminum silicates, polysaccharides and derivatives thereof, e.g. hyaluronic acid, xanthan gum, hydroxypropylmethylcellulose, particularly advantageously from the group of polyacrylates, preferably a polyacrylate from the group of so-called Carbopols, for example, Carbopol grades 980, 981, 1382, 2984, 5984, in each case individually or in combination.

Preferred cosmetic and/or pharmaceutical, especially dermatologically active preparations may also comprise active anti-inflammatory and/or redness- and/or itching-alleviating compounds (anti-irritants). All the active anti-inflammatory or redness- and/or itching-alleviating compounds which are suitable or usual for cosmetic and/or dermatological applications can be used here. Active anti-inflammatory and redness- and/or itching-alleviating compounds which are advantageously employed are steroidal anti-inflammatory substances of the corticosteroid type, such as hydrocortisone, dexamethasone, dexamethasone phosphate, methylprednisolone or cortisone, it being possible for the list to be extended by addition of further steroidal anti-inflammatories. Non-steroidal anti-inflammatories can also be employed. Those to be mentioned here by way of example are oxicams, such as piroxicam or tenoxicam; salicylates, such as aspirin, Disalcid, Solprin or fendosal; acetic acid derivatives, such as diclofenac, fenclofenac, indomethacin, sulindac, tolmetin, or clindanac; fenamates, such as mefenamic, meclofenamic, flufenamic or niflumic; propionic acid derivatives, such as ibuprofen, naproxen, benoxaprofen or pyrazoles, such as phenyl-butazone, oxyphenylbutazone, febrazone or azapropazone.

Alternatively, natural anti-inflammatory or redness- and/or itching-alleviating substances can be employed. Plant extracts, specific highly active plant extract fractions and highly pure active substances isolated from plant extracts can be employed. Extracts, fractions and active substances from camomile, aloe vera, Commiphora species, Rubia species, willow, rose-bay willow-herb, oats, and also pure substances, such as, inter alia, bisabolol, apigenin 7-glucoside, boswellic acid, phytosterols, glycyrrhizic acid, glabridin or licochalcone A, are particularly preferred. The preparations can also comprise mixtures of two or more active anti-inflammatory compounds. Bisabolol, boswellic acid, and also extracts and isolated highly pure active compounds from oats and Echinacea are particularly preferred for use as anti-inflammatory and redness- and/or itching-alleviating substances, and alpha-bisabolol and extracts and isolated highly pure active compounds from oats are especially preferred.

The amount of anti-irritants (one or more compounds) in the preparations is preferably 0.0001% to 20% by weight, with particular preference 0.0001% to 10% by weight, in particular 0.001% to 5% by weight, based on the total weight of the preparation.

Preferred cosmetic and/or pharmaceutical, especially dermatologically active preparations may advantageously also comprise moisture retention regulators. The following substances for example are used as moisture retention regulators (moisturizers): sodium lactate, urea, alcohols, sorbitol, glycerol, propylene glycol, aliphatic 1,2-diols with a C number of 5-10, collagen, elastin or hyaluronic acid, diacyl adipates, petrolatum, ectoin, urocanic acid, lecithin, pantheol, phytantriol, lycopene, algae extract, ceramides, cholesterol, glycolipids, chitosan, chondroitin sulphate, polyamino acids and polyamino sugars, lanolin, lanolin esters, amino acids, alpha-hydroxy acids (e.g. citric acid, lactic acid, malic acid) and derivatives thereof, sugars (e.g. inositol), alpha-hydroxy fatty acids, phytosterols, triterpene acids, such as betulinic acid or ursolic acid, algae extracts.

Preferred cosmetic and/or pharmaceutical, especially dermatologically active preparations may advantageously also comprise mono-, di- and oligosaccharides, such as, for example, glucose, galactose, fructose, mannose, fruit sugars and lactose.

Preferred cosmetic and/or pharmaceutical, especially dermatologically active preparations may advantageously also comprise plant extracts, which are conventionally prepared by extraction of the whole plant, but also in individual cases exclusively from blossom and/or leaves, wood, bark or roots of the plant. In respect of the plant extracts which can be used, reference is made in particular to the extracts which are listed in the table starting on page 44 of the 3rd edition of the Leitfaden zur Inhaltsstoffdeklaration kosmetischer Mittel [Manual of Declaration of the Constituents of Cosmetic Compositions], published by Industrieverband Körperpflegemittel und Waschmittel e.V. (IKW), Frankfurt. Extracts which are advantageous in particular are those from aloe, witch hazel, algae, oak bark, rose-bay willow-herb, stinging nettle, dead nettle, hops, camomile, yarrow, arnica, calendula, burdock root, horsetail, hawthorn, linden blossom, almond, pine needle, horse chestnut, sandalwood, juniper, coconut, mango, apricot, orange, lemon, lime, grapefruit, apple, green tea, grapefruit pip, wheat, oats, barley, sage, thyme, wild thyme, rosemary, birch, mallow, lady's smock, willow bark, restharrow, coltsfoot, hibiscus, ginseng and ginger root.

In this context, the extracts from aloe vera, camomile, algae, rosemary, calendula, ginseng, cucumber, sage, stinging nettle, linden blossom, arnica and witch hazel are particularly preferred. Mixtures of two or more plant extracts can also be employed. Extraction agents which can be used for the preparation of plant extracts mentioned are, inter alia, water, alcohols and mixtures thereof. In this context, among the alcohols lower alcohols, such as ethanol and isopropanol, but also polyhydric alcohols, such as ethylene glycol, propylene glycol and butylene glycol, are preferred, and in particular both as the sole extraction agent and in mixtures with water. The plant extracts can be employed both in pure and in diluted form.

Preferred cosmetic and/or pharmaceutical, especially dermatologically active preparations may in numerous cases advantageously comprise the following preservatives:
Preservatives which are preferably chosen here are those such as benzoic acid, its esters and salts, propionic acid and its salts, salicylic acid and its salts, 2,4-hexadienoic acid (sorbic acid) and its salts, formaldehyde and paraformaldehyde, 2-hydroxybiphenyl ether and its salts, 2-zincsulphidopyridine N-oxide, inorganic sulphites and bisulphites, sodium iodate, chlorobutanolum, 4-ethylmercuryl(II)-5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)urea, 4-chloro-m-cresol, 2,4,4'-trichloro-2'-hydroxydiphenyl ether, 4-chloro-3,5-dimethylphenol, 1,1'-methylene-bis(3-(1-hydroxymethyl-2,4-dioximidazolidin-5-yl)urea), poly(hexamethylene diguanide) hydrochloride, 2-phenoxyethanol, hexamethylenetetramine, 1-(3-chloroallyl)-3,5,7-triaza-1-azoniaadamantane chloride, 1-(4-chlorophenoxy)-1-(1H-imidazol-1-yl)-3,3-dimethyl-2-butanone, 1,3-bis-(hydroxymethyl)-5,5-dimethyl-2,4-imidazolidinedione, benzyl alcohol, octopirox, 1,2-dibromo-2,4-dicyanobutane, 2,2'-methylenebis(6-bromo-4-chlorophenol), bromochlorophene, mixture of 5-chloro-2-methyl-3(2H)-isothiazolinone and 2-methyl-3(2H)isothiazolinone with magnesium chloride and magnesium nitrate, 2-benzyl-4-chlorophenol, 2-chloroacetamide, chlorhexidine, chlorhexidine acetate, chlorhexidine gluconate, chlorhexidine hydrochloride, 1-phenoxypropan-2-ol, N-alkyl(C₁₂-C₂₂)trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxymethylurea, 1,6-bis(4-amidinophenoxy)-n-hexane and its salts, glutaraldehyde, 5-ethyl-1-aza-3,7-dioxabicyclo[3.3.0]octane, 3-(4-chlorophenoxy)-1,2-propanediol, hyamines, alkyl-(C₈-C₁₈)-dimethylbenzylammonium chloride, alkyl-(C₈-C₁₈)-dimethylbenzylammonium bromide, alkyl-(C₈-C₁₈)-dimethylbenzyl-ammonium saccharinate, benzyl hemiformal, 3-iodo-2-propynyl butylcarbamate, sodium hydroxymethylaminoacetate or sodium hydroxymethylaminoacetate.

In various cases it may also be advantageous to employ substances which are chiefly employed for inhibition of the growth of undesirable microorganisms on or in animal organisms in cosmetic and/or pharmaceutical, especially dermatologically active, preparations. In this respect, in addition to conventional preservatives, further active compounds which are worth mentioning, in addition to the large group of conventional antibiotics, are, in particular, the products relevant for cosmetics, such as triclosan, climbazol, octoxyglycerol, octopirox (1-hydroxy-4-methyl-6-(2,4,4-trimethylpentyl)-2(1H)-pyridone, 2-aminoethanol), chitosan, farnesol, glycerol monolaurate or combinations of the substances mentioned, which are employed, inter alia, against underarm odour, foot odour or dandruff formation.

Furthermore, cosmetic and/or pharmaceutical, especially dermatologically active preparations may also comprise substances having a cooling action. Individual active cooling compounds which are preferred for use are listed below. The skilled person is able to supplement the following list with a large number of further active cooling compounds; the active cooling compounds listed can also be employed in combination with one another: l-menthol, d-menthol, racemic menthol, menthone glycerol acetal (trade name: Frescolat^{®}MGA), menthyl lactate (trade name: Frescolat^{®}ML, menthyl lactate is preferably l-menthyl lactate, in particular l-menthyl l-lactate), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, 2-mercaptocyclodecanone, menthyl 2-pyrrolidin-5-onecarboxylate, 2,3-dihydroxy-p-menthane, 3,3,5-trimethylcyclohexanone glycerol ketal, 3-menthyl 3,6-di- and -trioxaalkanoates, 3-menthyl methoxyacetate, icilin.

Preferred active cooling compounds are: l-menthol, d-menthol, racemic menthol, menthone glycerol acetal (trade name: Frescolat^{®}MGA), menthyl lactate (preferably l-menthyl lactate, in particular l-menthyl l-lactate, trade name: Frescolat^{®}ML), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxtethyl menthyl carbonate, 2-hydroxypropyl menthyl carbonate, isopulegol.

Particularly preferred active cooling compounds are: 1-menthol, racemic menthol, menthone glycerol acetal (trade name: Frescolat^{®}MGA), menthyl lactate (preferably l-menthyl lactate, in particular l-menthyl l-lactate, trade name: Frescolat^{®}ML), 3-menthoxypropane-1,2-diol, 2-hydroxyethyl menthyl carbonate, 2-hydroxypropyl menthyl carbonate.

Very particularly preferred active cooling compounds are: l-menthol, menthone glycerol acetal (trade name: Frescolat^{®}MGA), menthyl lactate (preferably l-menthyl lactate, in particular l-menthyl l-lactate, trade name: Frescolat^{®}ML).

The use concentration of the active cooling compounds to be employed is, depending on the substance, preferably in the concentration range from 0.01% to 20% by weight, and more preferably in the concentration range from 0.1 % to 5% by weight, based on the total weight of the completed (ready-to-use) cosmetic or pharmaceutical preparation.

The following examples are intended to illustrate the present invention without restricting the scope of this invention and particularly the scope of the claims. All amounts quoted, proportions and percentages are, unless indicated otherwise, based on the weight and the total amount or on the total weight of the preparations. When these preparations are irradiated with UV radiation they do not fluoresce whereas the preparations without the fluorescent quencher (troxerutin) fluoresce strongly.

### Examples

### Fluorescence.

By way of example, comparative observations between preparations containing disodium phenyl dibenzimidazole tetrasulfonic acid and preparations containing mixtures of disodium phenyl dibenzimidazole tetrasulfonic acid and troxerutin are listed below:

### Example 1

| **Part** | **Raw materials** | **INCI name** | **A % (w/w)** | **B% (w/w)** |
|---|---|---|---|---|
| A | Emulsiphos | Potassium Cetyl Phosphate (and Hydrogenated Palm Glycerides) | 1.50 | 1.50 |
| | Lanette O | Cetearyl Alcohol | 1.50 | 1,50 |
| | Copherol 1250 | Tocopherylacetate | 0.50 | 0.50 |
| | Dow Corning 246 Fluid | Cyclohexasiloxane | 2.00 | 2.00 |
| | Tegosoft TN | C 12-15 Alkyl Benzoate | 5.00 | 5.00 |
| | Neo Heliopan^{®} AV | Ethyl Methoxycinnamate | 7.50 | 7.50 |
| | Neo Heliopan^{®} E1000 | Isoamyl p-Methoxycinnamate | 7.50 | 7.50 |
| | Carbopol ETD 2050 | Carbomer | 0.20 | 0.20 |
| | Keltrol T | Xanthan Gum | 0.20 | 0.20 |
| | EDETA B fl. | Tetrasodium EDTA | 0.10 | 0.10 |
| B | Distilled Water | Water (Aqua) | Ad 100 | Ad 100 |
| | Neo Heliopan^{®} AP | Disodium Phenyl Dibenzimidazole Tetrasulfonate | 2.00 | 2.00 |
| | Troxerutin | Troxerutin | 0.00 | 0.20 |
| | Triethanolamine | Triethanolamine | qs | Qs |
| | | | | |
| C | Fragrance | Parfum | 0.40 | 0.40 |

Formula A when applied to human skin fluoresces strongly under UVA irradiation, Sample B does not fluoresce under the same conditions.

### Example 2

| **Part** | **Raw materials** | **INCI name** | **A % (w/w)** | **B% (w/w)** |
|---|---|---|---|---|
| A | Emulsiphos | Potassium Cetyl Phosphate (and Hydrogenated Palm Glycerides) | 1.50 | 1.50 |
| | Lanette O | Cetearyl Alcohol | 1.50 | 1,50 |
| | Copherol 1250 | Tocopherylacetate | 0.50 | 0.50 |
| | Dow Corning 246 Fluid | Cyclohexasiloxane | 2.00 | 2.00 |
| | Tegosoft TN | C 12-15 Alkyl Benzoate | 5.00 | 5.00 |
| | Neo Heliopan^{®} AV | Ethyl Methoxycinnamate | 7.50 | 7.50 |
| | Neo Heliopan^{®} E1000 | Isoamyl p-Methoxycinnamate | 10.50 | 10.50 |
| | Carbopol ETD 2050 | Carbomer | 0.20 | 0.20 |
| | Keltrol T | Xanthan Gum | 0.20 | 0.20 |
| | EDETA B fl. | Tetrasodium EDTA | 0.10 | 0.10 |
| | | Titanium Dioxide | 3.00 | 3.00 |
| | | | | |
| B | Distilled Water | Water (Aqua) | Ad 100 | Ad 100 |
| | Neo Heliopan^{®} AP | Disodium Phenyl Dibenzimidazole Tetrasulfonate | 2.00 | 2.00 |
| | Troxerutin | Troxerutin | 0.00 | 0.20 |
| | Sodium Hydroxide | Sodium Hydroxide | qs | Qs |
| C | Fragrance | Parfum | 0.40 | 0.40 |

Formula A when applied to human skin fluoresces strongly under UVA irradiation, Sample B does not fluoresce under the same conditions.

### Example 3

| **Part** | **Raw materials** | **INCI name** | **A % (w/w)** | **B% (w/w)** |
|---|---|---|---|---|
| **A** | Emulsiphos | Potassium Cetyl Phosphate (and Hydrogenated Palm Glycerides) | 1.50 | 1.50 |
| | Lanette O | Cetearyl Alcohol | 1.50 | 1,50 |
| | Copherol 1250 | Tocopherylacetate | 0.50 | 0.50 |
| | Dow Corning 246 Fluid | Cyclohexasiloxane | 2.00 | 2.00 |
| | Tegosoft TN | C 12-15 Alkyl Benzoate | 5.00 | 5.00 |
| | Neo Heliopan^{®} AV | Ethyl Methoxycinnamate | 7.50 | 7.50 |
| | Neo Heliopan^{®} E1000 | Isoamyl p-Methoxycinnamate | 7.50 | 7.50 |
| | Carbopol ETD 2050 | Carbomer | 0.20 | 0.20 |
| | Keltrol T | Xanthan Gum | 0.20 | 0.20 |
| | EDETA B fl. | Tetrasodium EDTA | 0.10 | 0.10 |
| | | | | |
| B | Distilled Water | Water (Aqua) | Ad 100 | Ad 100 |
| | Glycerin | Glycerin | 4.70 | 4.70 |
| | Phenonip | Phenoxyethanol, Methylparaben, E-thylparaben, Butylparaben, Propylparaben, Isobutylparaben | 0.80 | 0.80 |
| | Neo Heliopan^{®} AP | Disodium Phenyl Dibenzimidazole Tetrasulfonate | 2.00 | 2.00 |
| | Troxerutin | Troxerutin | 0.00 | 0.5 |
| | Triethanolamine | Triethanolamine | qs | Qs |
| | | | | |
| C | Fragrance | Parfum | 0.40 | 0.40 |

Formula A when applied to human skin fluoresces strongly under UVA irradiation, Sample B does not fluoresce under the same conditions.

### Manufacturing procedure for examples 1-3.

Part A. Mix all ingredients except Keltrol and Carbopol and heat up to 85°C until all ingredients are completely dissolved. Add Keltrol and Carbopol and homogenise.
Part B. Mix all ingredients together and heat to 85°C, then add to Part A. with stirring. Homogenise and cool to ambient temperature.
Part C. Add to parts A and B at 30°C with stirring. Homogenise.

### Formulation Examples.

### Formulation Example 1 Sunscreen soft cream (O/W), in-vitro SPF 5, water resistant

| Part | Raw Materials | INCI Name | % (wt.) |
|---|---|---|---|
| A | Emulsiphos | Potassium Cetyl Phosphate, Hydrogenated Palm Glycerides | 1.50 |
| | Cutina GMS / V | Glyceryl Stearate | 2.00 |
| | Copherol 1250 | Tocopheryl Acetate | 0.50 |
| | Lanette 16 | Cetyl Alcohol | 1.00 |
| | Tegosoft TN | C 12-15 Alkyl Benzoate | 24.00 |
| | Prisorine 3505 | Isostearic Acid | 1.00 |
| | Heo Heliopan® 357 | Butyl Methoxydibenzolymethane | 3.00 |
| | Neo Heliopan® 303 | Octocrylene | 6.00 |
| B | Water, dist. | Water (Aqua) | Ad 100 |
| | Neo Heliopan® AP | Disodium Phenyl Dibenzimidazole Tetrasulfonate | 2.0 |
| | Troxerutin | Troxerutin | 0.5 |
| | Sodium Hydroxide | Sodium Hydroxide | qs |
| | EDETA B liq. | Tetrasodium EDTA | 0.20 |
| | Glycerol, 99% | Glycerin | 3.00 |
| | Phenoxyethanol | Phenoxyethanol | 0.70 |
| | Solbrol M | Methylparaben | 0.20 |
| | Solbrol P | Propylparaben | 0.10 |
| | Carbopol ETD 2050 | Carbomer | 0.20 |
| C | Perfume oil | Parfum (Fragrance) | 0.30 |

### Manufacturing procedure.

Part A: Heat to about 85°C.
Part B: Weigh in raw materials without Carbopol. Disperse Carbopol therein using Ultra Turrax. Heat to about 85°C. Add B to A. and then homogenise while hot (Ultra Turrax). Leave to cool with stirring.
Part C: Add to A/B at 30°C or less with stirring

### Formulation Example 2 Sunscreen lotion (O/W), in-vitro SPF 20

| Part | Raw Materials | INCI Name | % (wt.) |
|---|---|---|---|
| A | Emulsiphos | Potassium Cetyl Phosphate, Hydrogenated Palm Glycerides | 1.50 |
| | Cutina GMS / V | Glyceryl Stearate | 2.00 |
| | Copherol 1250 | Tocopheryl Acetate | 0.50 |
| | Lanette 16 | Cetyl Alcohol | 1.00 |
| | Tegosoft TN | C 12-15 Alkyl Benzoate | 10.60 |
| | Prisorine 3505 | Isostearic Acid | 1.00 |
| | Neo Heliopan 357 | Butyl Methoxydibenzolymethane | 2.00 |
| | Neo Heliopan® AV | Ethylhexyl Methoxycinnamate | 5.00 |
| | EDETA BD | Disodium EDTA | 0.10 |
| | Carbopol ETD 2050 | Carbomer | 0.20 |
| B | Water, dist. | Water (Aqua) | qs |
| | Glycerol, 99% | Glycerin | 3.00 |
| | Dragocid Liquid | Phenoxyethanol, Methylparaben, Ethylparaben, Butylparaben, Propylparaben, Isobutylparaben | 0.80 |
| C | Triethanolamine | Triethanolamine | qs |
| | Neo Heliopan® AP 22% strength solution neutralised with Triethanolamine | Disodium Phenyl Dibenzimidazole Tetrasulfonate | 9.1 |
| | Neo Heliopan® Hydro, 30% strength solution neutralised with Triethanolamine | Phenylbenzimidazole Sulfonic Acid | 3.34 |
| | Troxerutin | Troxerutin | 0.5 |
| D | Perfume oil | Parfum (Fragrance) | 0.30 |

### Manufacturing procedure.

Part A: Heat to about 85°C.
Part B: Weigh in raw materials without Carbopol. Disperse Carbopol therein using Ultra Turrax. Heat to about 85°C. Add B to A.
Part C: Immediately add to A/B and then homogenise while hot (Ultra Turrax). Leave to cool with stirring.
Part D: Add and stir in.

### Formulation Example 3 Low oil content Sunscreen milk (O/W), in-vitro SPF 25

| Part | Raw Materials | INCI Name | % (wt.) |
|---|---|---|---|
| A | Tegin M | Glyceryl Stearate | 2.50 |
| | Tagat S | PEG-30 Glyceryl Stearate | 1.95 |
| | Lanette O | Cetearyl Alcohol | 2.20 |
| | Copherol 1250 | Tocopheryl Acetate | 0.50 |
| | Phenonip | Phenoxyethanol (and) methylparaben (and) Butylparaben (and) ethyl-paraben (and) Propylparaben | 0.15 |
| | Neo Heliopan® AV | Ethylhexyl Methoxycinnamate | 5.00 |
| | Neo Heliopan® 303 | Octocrylene | 5.00 |
| | Neo Heliopan® 357 | Butyl Methoxydibenzolymethane | 2.00 |
| | EDETA BD | Disodium EDETA | 0.10 |
| B | Water, dist. | Water (Aqua) | Ad 100 |
| | Neo Heliopan® Hydro | Phenylbenzimidazole Sulfonic Acid, | 3.3 |
| | Neo Heliopan® AP, | Disodium Phenyl Dibenzimidazole Tetrasulfonate | 2.20 |
| | Troxerutin | Troxerutin | 0.5 |
| | 1,2-Propylene glycol | Propylene Glycol | 2.00 |
| | Phenonip | Phenoxyethanol (and) Methylparaben (and) Butylparaben (and) Ethylparaben (and) Propylparaben | 0.30 |
| | NaOH, 10% strength | Sodium Hydroxide | 2.2 |
| C | Water, dist. | Water (Aqua) | 19.00 |
| | Carbopol 2050 | Carbomer | 0.40 |
| | NaOH, 10% strength | Sodium Hydroxide | Qs |
| D | Perfume oil | Parfum (Fragrance) | 0.30 |

### Manufacturing procedure.

Part A: Heat to 80-85°C.
Part B: Heat to 80-85°C, Add part B to part A with stirring.
Part C: Disperse Carbopol into the water and neutralise with NaOH, with stirring. Add part C at about 60°C with stirring. Allow to cool to RT (room temperature).
Part D: Add and stir.

### Formulation Example 4 Sunscreen lotion (O/W), in-vitro SPF 18

| Part | Raw Materials | INCI Name | % (wt.) |
|---|---|---|---|
| A | Eumulgin VL 75 | Lauryl Glucoside (and) Polyglyceryl-2-Dipolyhydroxystearate (and) Glycerin | 3.00 |
| | Tegosoft TN | C12-25 Alkyl Benzoate | 20.00 |
| | Copherol 1250 | Tocopheryl Acetate | 0.50 |
| | Perfume oil | Parfum (Fragrance) | 0.20 |
| | Neo Heliopan® 357 | Butyl Methoxydibenzolymethane | 2.00 |
| | Neo Heliopan® E1000 | Isoamyl p-Methoxycinnamate | 5.00 |
| | Neo Heliopan® 303 | Octocrylene | 5.00 |
| | Carbopol 2050 ETD | Carbomer | 0.35 |
| | Pemulen TR-1 | Acrylates/C10-30 Alkyl Acrylate Cross-polymer | 0.15 |
| | EDETA BD | Disodium EDTA | 0.10 |
| B | Water, dist. | Water (Aqua) | Ad 100 |
| | Glycerol, 99% | Glycerin | 5.00 |
| | Dragocid Liquid | Phenoxyethanol, Methylparaben, Ethylparaben, Butylparaben, Propylparaben, Isobutylparaben | 0.80 |
| | Neo Heliopan® Hydro | Phenylbenzimidazole Sulfonic Acid | 2.00 |
| | Neo Heliopan® AP | Disodium Phenyl Dibenzimidazole Tetrasulfonate | 1.10 |
| | Troxerutin | Troxerutin | 0.3 |
| | Amino Methyl Propanol | Amino Methyl Propanol | 4.00 |
| C | Perfume oil | Parfum (Fragrance) | 0.30 |

### Manufacturing procedure.

Part A: Dissolve the solids in the oils and liquid UV filters (heating to about 70°C). Allow to cool to about 30°C, add the remaining constituents apart from Carbopol and Pemulen and mix at room temperature (stir for about 5 minutes). Stir in Carbopol and Pemulen.
Part B: Add water and glycerin, then disperse Neo Heliopan® Hydro with vigorous stirring and heating to 70°C, add Amino Methyl Propanol until all of the Neo Heliopan® Hydro, Neo Heliopan® AP has been dissolved and add all of part B to part A with stirring. Stir for about 60 minutes with cooling and homogenise using the Ultra Turrax.
Part C: Stir in at ambient temperature.

### Formulation Example 5 Sunscreen cream (W/O), in-vitro SPF 10, water resistant

| Part | Raw Materials | INCI Name | % (wt.) |
|---|---|---|---|
| A | Dehymuls PGPH | Polyglyceryl-2 Dipolyhydroxystearate | 5.00 |
| | Copherol 1250 | Tocopheryl Acetate | 0.50 |
| | Permulgin 3220 | Ozokerite | 0.50 |
| | Aluminium stearate | Aluminium Stearate | 0.50 |
| | Tegosoft TN | C12-15 Alkyl Benzoate | 25.00 |
| | Neo Heliopan® 357 | Butyl Methoxydibenzolymethane | 2.00 |
| | EDETA BD | Disodium EDTA | 0.10 |
| B | Water, dist. | Water (Aqua) | Ad 100 |
| | Glycerol, 99% | Glycerin | 4.00 |
| | Dragocid Liquid | Phenoxyethanol, Methylparaben, Ethylparaben, Butylparaben, Propylparaben, Isobutylparaben | 1.00 |
| | Neo Heliopan® Hydro, 22% strength solution neutralised with Triethanolamine | Phenylbenzimidazole Sulfonic Acid | 22.3 |
| | Neo Heliopan® AP 22% strength solution neutralised with Triethanolamine | Disodium Phenyl Dibenzimidazole Tetrasulfonate | 6.0 |
| | Troxerutin | Troxerutin | 0.3 |
| | Triethanolamine | Triethanolamine | qs |
| | Magnesium sulfate | Magnesium Sulfate | 0.50 |
| C | Perfume oil | Parfum (Fragrance) | 0.30 |

### Manufacturing procedure.

Part A: Heat to about 85°C.
Part B: Heat to about 85°C. Add B to A. Allow to cool with stirring then homogenise.
Part C: Stir in at ambient temperature.

### Formulation Example 6 Sunscreen softcream (W/O), in-vitro SPF 50

| Part | Raw Materials | INCI Name | % (wt.) |
|---|---|---|---|
| A | Dehymuls PGPH | Polyglyceryl-2 Dipolyhydroxystearate | 5.00 |
| | Copherol 1250 | Tocopheryl Acetate | 0.50 |
| | Permulgin 3220 | Ozokerite | 0.50 |
| | Zinc stearate | Zinc Stearate | 0.50 |
| | Tegosoft TN | C12-15 Alkyl Benzoate | 10.00 |
| | Neo Heliopan®BB | Benzophenone-3 | 3.00 |
| | Neo Heliopan® HMS | Homosalate | 5.00 |
| | Neo Heliopan® 303 | Octocrylene | 5.00 |
| | Neo Heliopan® OS | Ethylhexyl Salicylate | 5.00 |
| | Uvinul T-150 | Ethylhexyl Triazone | 3.00 |
| | Zinc oxide neutral | Zinc Oxide | 5.00 |
| B | Water, dist. | Water (Aqua) | Ad 100 |
| | EDETA BD | Disodium EDTA | 0.10 |
| | Glycerol, 99% | Glycerin | 4.00 |
| | Dragocid Liquid | Phenoxyethanol, Methylparaben, Ethylparaben, Butylparaben, Propylparaben, Isobutylparaben | 1.00 |
| | Neo Heliopan® Hydro, 15% strength solution neutralised with Sodium Hydroxide | Phenylbenzimidazole Sulfonic Acid | 10.00 |
| | Neo Heliopan® AP 10% strength solution neutralised with Sodium Hydroxide | Disodium Phenyl Dibenzimidazole Tetrasulfonate | 12.0 |
| | Troxerutin | Troxerutin | 0.5 |
| | Sodium Hydroxide | Sodium Hydroxide | qs |
| | Magnesium sulfate | Magnesium Sulfate | 0.50 |
| C | Parfume oil | Parfum (Fragrance) | 0.20 |

### Manufacturing procedure.

Part A: Heat to about 85°C.
Part B: Heat to about 85°C (without zinc oxide; disperse zinc oxide therein using the Ultra Turrax). Add B to A. Allow to cool with stirring.
Part C: Add and then homogenise.

### Formulation Example 7 Day care cream with broad spectrum UV protection

| Part | Raw Materials | INCI Name | % (wt.) |
|---|---|---|---|
| A | Emulgade PL 68/50 | Cetearyl Glycoside (and) Cetearyl Alcohol | 4.50 |
| | Cetiol PGL | Hexyldecanol (and) Hexyldecyl Laurate | 8.00 |
| | Myritol 331 | Cocoglycerides | 8.00 |
| | Copherol 1250 | Tocopheryl Acetate | 0.50 |
| | Neo Heliopan® 303 | Octocrylene | 3.00 |
| | Neo Heliopan® OS | Ethylhexyl Salicylate | 5.00 |
| B | Water, dist. | Water (Aqua) | Qs |
| | Aqueous mixture of 30% Neo Heliopan® Hydro and 22% Neo Heliopan® AP neutralised with Triethanolamine | Phenylbenzimidazole Sulfonic Acid, Disodium Phenyl Dibenzimidazole Tetrasulfonate | 11.00 |
| | Troxerutin | Troxerutin | 0.3 |
| | Triethanolamine | Triethanolamine | Qs |
| | Glycerol | Glycerin | 3.00 |
| | Dragocide Liquid | Phenoxyethanol (and) Methylparaben (and) Butyparaben (and) Ethyparaben (and) Propylparaben | 0.50 |
| C | Water, dist. | Water (Aqua) | 25.00 |
| | Carbopol ETD 2050 | Carbomer | 0.20 |
| | Triethanolamine | Triethanolamine | Qs |
| D | Perfume oil | Parfum (Fragrance) | 0.30 |

### Manufacturing procedure.

Part A: Heat to 80°C.
Part B: Heat to 80°C Add to part A with stirring.
Part C: Disperse Carbopol in water and neutralise with sodium hydroxide solution. Add to part A/B at about 55°C.
Part D: Stir in at ambient temperature.

### Formulation Example 8 Sunscreen spray in-vitro SPF 20

| Part | Raw Materials | INCI Name | % (wt.) |
|---|---|---|---|
| A | Water, demin. | Water (Aqua) | 62.1 |
| | Glycerol, 99% | Glycerin | 4.00 |
| | Hydrolite 5 | 1,2-Pentylene Glycol | 5.00 |
| | D-Panthenol | Panthenol | 0.50 |
| | Lara Care A-200 | Galactoarabinan | 0.25 |
| B | Baysilone oil M 10 | Dimethicone | 1.00 |
| | Edeta BD | Disodium EDTA | 0.10 |
| | Copherol 1250 | Tocopheryl Acetate | 0.50 |
| | Cetiol OE | Dicaprylyl Ether | 3.00 |
| | Neo Heliopan® HMS | Homosalate | 5.00 |
| | Neo Heliopan® AV | Ethylhexyl Methoxycinnamate | 6.00 |
| | Neo Heliopan® 357 | Butyl Methoxydibenzoylmethane | 1.00 |
| | Tinosorb® S | Bis Ethylhexyloxyphenol Methoxyphenyl Triazine | 2.00 |
| | Alpha-Bisabolol | Bisabolol | 0.10 |
| | Pemulen TR-2 | Acrylates/C10-30 Alkyl Acrylate Crosspolymer | 0.25 |
| C | Phenoxyethanol | Phenoxyethanol | 0.70 |
| | Solbrol M | Methylparaben | 0.20 |
| | Solbrol P | Propylparaben | 0.10 |
| | Neo Heliopan® Hydro | Phenylbenzimidazole Sulfonic Acid | 2.00 |
| | Neo Heliopan® AP | Disodium Phenyl Dibenzimidazole Tetrasulfonate | 1.50 |
| | Troxerutin | Troxerutin | 0.5 |
| | Arginine | Arginine | qs |
| D | Perfume oil | Fragrance (Parfum) | 0.20 |

### Manufacturing procedure.

Part A: Dissolve Lara Care A-200 into the other constituents of part A with stirring.
Part B: Weigh in all raw materials (without Pemulen) and dissolve the crystalline substances with heating. Disperse Pemulen therein. Add part B to part A then homogenise for 1 minute.
Part C: Stirr in the ingredients until all have dissolved and add part C+D then homogenise again for 1-2 minutes using the Ultra Turrax.

The pH of the formulation is 6.2

### Formulation Example 9 Sunscreen hydrodispersion gel (balm)

| Part | Raw Materials | INCI Name | % (wt.) |
|---|---|---|---|
| A | Water, dist. | Water (Aqua) | Ad 100 |
| | Carbopol Ultrez 21 | Acrylates/C10-30 Alkyl Acrylate Crosspolymer | 1.00 |
| | Triethanolamine | Triethanolamine | 1.20 |
| | | | |
| B | Neo Heliopan® AP, 22% strength solution neutralised with Triethanolamine | Disodium Phenyl Dibenzimidazole Tetrasulfonate | 10.0 |
| | Troxerutin | Troxerutin | 0.5 |
| | Triethanolamine | Triethanolamine | qs |
| | | | |
| C | Neo Heliopan® E1000 | Isoamyl p-I Methoxycinnamate | 3.00 |
| | Neo Heliopan® 357 | Butyl Methoxydibenzoylmethane | 2.00 |
| | Isopropyl myristate | Isopropyl Myristate | 4.00 |
| | Baysilone oil PK 20 | Phenyl Trimethicone | 3.00 |
| | Dragocide Liquid | Phenoxyethanol (and) Methylparaben (and) Butyparaben (and) Ethyparaben (and) Propylparaben | 0.50 |
| | Perfume oil | Parfum (Fragrance) | 0.30 |
| | Edeta BD | Disodium EDTA | 0.10 |
| | Alpha Bisabolol | Alpha Bisabolol | 0.10 |

### Manufacturing procedure.

Part A: Disperse Carbopol in water and neutralise with triethanolamine solution.
Part B: Add to part A with stirring.
Part C: Dissolve crystalline constituents in the other raw materials of part C with warming (max.40°C) and add to part A/B. Stir well and then homogenise. (Homozenta).

### Formulation Example 10 Hair Conditioner with UV filters

| Part | Raw Materials | INCI Name | % (wt.) |
|---|---|---|---|
| A | Renex PEG 6000 | PEG-150 | 2.5 |
| | Hair Conditioner Base | Cetyl Alcohol, Behentrimonium Chloride, Triticum Vulgare (Wheat) Bran Extract, Linoleic Acid | 3.0 |
| | PCL-Solid | Stearyl Heptanoate, Stearyl Caprylate | 0.50 |
| | Dow Corning 5200 | Laurylmethicone Copolyol | 0.50 |
| B | Natrosol 250 HR | Hydroxyethylcellulose | 0.10 |
| | Water, dist. | Water (Aqua) | 87.07 |
| | Neo Heliopan® AP | Disodium Phenyl Dibenzimidazole Tetrasulfonate | 0.50 |
| | Troxerutin | Troxerutin | 0.50 |
| | Amino Methyl propanol | Amino Methyl propanol | qs |
| | Nipagin M | Methylparaben | 0.30 |
| C | Dow Corning 949 Cationic Emulsion | Amodimethicone, Cetrimonium Chloride, Trideceth-12 | 2.00 |
| | Perfume oil | Parfum (Fragrance) | 0.80 |

### Manufacturing procedure.

Part A: Heat to 80°C.
Part B: Swell Natrosol in water, Neo Heliopan® AP and Amino Methyl Propanol, add Nipagin M and heat to 80°C. Add to part A with stirring and emulsify. Cool down with stirring.
Part C: Add at 35°C and cool to RT with stirring.

### Formulation Example 11 Broad Spectrum Aqueous Gel in-vitro SPF 14.0

| Part | Ingredients | INCI-Name | % (wt.) |
|---|---|---|---|
| A | Demineralised Water | Water (Aqua) | 59.20 |
| | Amaze XT | Dehydroxanthan Gum | 1.00 |
| B | Demineralised Water | Water (Aqua) | 15.00 |
| | Neo Heliopan® Hydro | Phenylbenzimidazole Sulfonic Acid | 3.00 |
| | Neo Heliopan® AP | Disodium Phenyl Dibenzimidazole Tetrasulfonate | 3.00 |
| | Troxerutin | Troxerutin | 0.75 |
| | Triethanolamine | Triethanolamine | qs |
| C | Symdiol 68 | 1,2-Hexanediol and 1,2-Octanediol | 0.50 |
| | Hydrolite 5 | Pentylene Glycol | 2.00 |
| | Dragoderm | Glycerin, Triticum Vulgare (Wheat) Gluten, Water (Aqua) | 2.00 |
| D | Dow Corning 193 | PEG -12 Dimethicone | 1.00 |
| | Ethanol 96% | SD-Alcohol 39-C | 10.00 |
| | Perfume oil | Fragrance | 0.10 |

### Manufacturing procedure.

Part A: Stir Amaze XT into the water with stirring until it is swollen and a gel has formed
Part B: Stir the ingredients together and add to Part A and then add Parts C with stirring until uniform and then add Part D with gentle stirring.

### Formulation Examples 12 Water resistant Broad Spectrum O/W emulsions in-vitro SPF 50+

| Part | Ingredients | INCI | A % (wt.) | B % (wt.) | C % (wt.) |
|---|---|---|---|---|---|
| A | Emulsiphos | Potassium Cetyl Phosphate, Hydrogenated Palm Glycerides | 3.50 | 3.50 | 3.50 |
| | Lanette O | Cetearylalcohol | 1.00 | 1.00 | 1.00 |
| | Neo Heliopan® HMS | Homosalate | 5.00 | 5.00 | 5.00 |
| | Neo Heliopan® 303 | Octocrylene | 10.00 | 10.00 | 10.00 |
| | Neo Heliopan® OS | Ethylhexyl Salicylate | 5.00 | 5.00 | 5.00 |
| | Neo Heliopan® 357 | Butyl Methoxydibenzoylmethane | 5.00 | 4.50 | 5.00 |
| | Eusolex T2000 | Titanium Dioxide, Alumina, Simethicone | 5.00 | 5.00 | 5.00 |
| | Tinosorb S | Bis Ethylhexyloxyphenol Methoxyphenyl Triazine | 0.00 | 4.50 | 3.00 |
| | Abil Wax 9801 | Cetyl Dimethicone | 1.00 | 1.00 | 1.00 |
| | Silcare Silicone 41 M65 | Stearyl Dimethicone | 1.00 | 1.00 | 1.00 |
| | Baysilone oil PK 20 | Phenyl Trimethicone | 2.00 | 2.00 | 2.00 |
| | Isoadipat | Diisopropyladipate | 2.00 | 2.00 | 2.00 |
| | Tocopherylacetat | Tocopheryl Acetate | 0.50 | 0.50 | 0.50 |
| | Antaron V216 | VP/Hexadecene Copolymer | 0.50 | 0.50 | 0.50 |
| | EDTA BD | Disodium EDTA | 0.10 | 0.10 | 0.10 |
| | Keltrol T | Xanthan Gum | 0.50 | 0.50 | 0.50 |
| B | Water dem | Water (Aqua) | Ad 100 | Ad 100 | Ad 100 |
| | Troxerutin | Troxerutin | 1.0 | 1.0 | 1.00 |
| | Neo Heliopan® AP | Disodium Phenyl Dibenzimidazole Tetrasulfonate | 2.00 | 1.50 | 1.00 |
| | Phenonip | Phenoxyethanol (and) Methylparaben (and) Butyparaben (and) Ethyparaben (and) Propylparaben | 0.70 | 0.70 | 0.70 |
| | Arginine | Arginine | 2.20 | 1.23 | 1.72 |
| | Lara Care A-200 | Galactoarabinan | 0.25 | 0.25 | 0.25 |
| | Hydrolite 5 | Pentylene Glycol | 3.00 | 3.00 | 3.00 |
| C | Fragrance | Fragrance (parfum) | 0.30 | 0.30 | 0.30 |

### Manufacturing procedure.

Part A: Heat all components except for the Xanthan Gum and TiO₂ to 85°C. Then add Xanthan Gum and TiO₂ and homogenise.
Part B: Heat all components to 85°C and add to Part A with stirring, stir to room temperature.
Part C: Add Part C to Parts A & B and homogenise.

### Formulation Examples 13 Sunspray O/W exp. SPF 20

| Part | Ingredients | INCI | % (wt.) |
|---|---|---|---|
| A | Dracorin GOC | Glyceryl Oleate Citrate, Caprylic/Capric Triglyceride | 2.00 |
| | Neo Heliopan HMS | Homosalate | 7.00 |
| | Neo Heliopan 357 | Butyl Methoxydibenzoylmethane | 4.00 |
| | Neo Heliopan OS | Ethylhexyl Salicylate | 5.00 |
| | Isoadipat | Diisopropyl Adipate | 6.00 |
| | Corapan TQ | Diethylhexyl 2,6 Naphthalate | 3.00 |
| | Edeta BD | Disodium EDTA | 0.10 |
| | Vitamin E Acetat | Tocopheryl Acetate | 0.50 |
| | Baysilone Oil M 10 | Dimethicone | 1.00 |
| | Alpha-Bisabolol | Bisabolol | 0.10 |
| | Pemulen TR 2 | Acrylates/ C10-30 Acrylates Copolymer | 0.25 |
| | Perfume | Fragrance (parfum) | 0.25 |
| B | Deionised water | Water (Aqua) | Ad 100 |
| | Glycerin 99% | Glycerin | 4.00 |
| | Butylenglycol | Butylene Glycol | 5.00 |
| | Dragocid Liquid | Phenoxyethanol, Methylparaben, Ethylparaben, Butylparaben, Propylparaben, Isobutylparaben | 0.80 |
| | Neo Heliopan® Hydro | Phenylbenzimidazole Sulfonic Acid | 2.00 |
| | Troxerutin | Troxerutin | 1.0 |
| | Neo Heliopan® AP | Disodium Phenyl Dibenzimidazole Tetrasulfonate | 2.00 |
| | Tris-Hydroxyaminomethane | Tris-Hydroxyaminomethane | 0.47 |
| C | Fragrance | Fragrance (parfum) | 0.30 |

### Manufacturing procedure.

Part A: Dissolve the Neo Heliopan 357 in the other components of phase A (except for Pemulen and EDTA) by heating up to 50°C. The add Xanthan Gum and TiO₂ and homogenise.
Part B: Add to Part A without stirring, then start emulsifying.
Part C: Add Part C to Parts A & B while homogenising.

### Formulation Example 14 Clear Hair Shampoo with UV-Protection

| Part | Ingredients | INCl-Name | % (wt.) |
|---|---|---|---|
| A | Demineralised Water | Water (Aqua) | Ad 100 |
| | Merquat 550 | Polyquaterium-7 | 0.50 |
| B | Neo Heliopan® AP | Disodium Phenyl Dibenzimidazole Tetrasulfonate | 1.00 |
| | Troxerutin | Troxerutin | 0.3 |
| | Amino Methyl Propanol | Amino Methyl Ppropanol | 0.6 |
| C | Genapol LRO Liquid | Sodium Laureth Sulfate | 30.00 |
| | Tego Betain F 50 | Cocoamidopropyl Betaine | 5.00 |
| | Antil 141 | Propylene Glycol, PEG-55 Propylene Glycol Dioleate | 0.80 |
| | Dragocide Liquid | Phenoxyethanol, Methylparaben, Ethylparaben, Butylparaben, Propylparaben, Isobutylparaben | 0.80 |
| | D-Panthenol 75 L | Panthenol | 1.00 |
| | Extrapone® Lime | Propylene Glycol, Water (Aqua), Citrus Aurantifolia (Lime) Juice | 1.00 |
| | Sodium Chloride | Sodium Chloride | 0.70 |
| | Perfume | Fragrance | 0.40 |

### Manufacturing procedure.

Part A: Dissolve Merquat in water with stirring
Part B: Add Neo Heliopan Hydro and neutralise with Amino Methyl Propanol, dissolve until a clear solution has formed.
Part C: Add the ingredients to part AB as listed and stir until a uniform solution has formed. The viscosity can be adjusted by the amount of sodium chloride.

The pH of the resulting formulation was in the range of 5.2 - 5.5

### Other Formulation Examples:

### 1. O/W Emulsions:, SPF > 20

| **RAW MATERAL NAME (MANUFACTURER)** | INCI | | | | | | | | | | | |
|---|---|---|---|---|---|---|---|---|---|---|---|---|
| | | **1** | **2** | **3** | **4** | **5** | **6** | **7** | **8** | **9** | **10** | **11** |
| **Emulsifier** | | | | | | | | | | | | |
| Emulsiphos (Symrise) | Potassium Cetyl Phosphate, Hydrogenated Palm Glycerides | | | | 2.0 | | | | | | | |
| Dracorin CE | Glyceryl Stearate Citrate | 1.0 | 1.0 | | | | | | | | | |
| Dracorin GOC (Symrise) | Glyceryl Oleyl Citrate | | | | | 4.0 | | | | | | |
| | Polyglyceryl 2-Dipolyhydroxystearate | 0.25 | 0.25 | | | | | | | | | |
| | Cetearyl Alcohol, Peg-40 Castor Oil, Sodium Cetearyl Sulfate | | | | | | | 3.75 | | | | |
| | PEG-30 Dipolyhydroxystearate | | | | | | | 1.0 | | | | |
| | Polyglyceryl-3 Methylglucose Distearate | | | | | | | | 2.0 | | | |
| | Sorbitan Stearate | | | | 0.5 | | | | 1.0 | | | |
| | Glyceryl Stearate SE | | | | | | | 1.5 | | | | |
| | Glyceryl Stearate | | | 2.5 | 1.0 | | 4.0 | | | | 4.0 | |
| | Isostearic Acid | | | | | | 1.0 | | | | | |
| | Stearic Acid | | | | 1.0 | | | | | 4.0 | | 0.5 |
| | PEG 40 Stearate | | | 1.0 | | | | | | 1.0 | | |
| | PEG 100 Stearate | | | | | | | | | | 2.0 | 0.5 |
| | Potassium Cetyl Phosphate | | | | | | 2.0 | | | | 0.5 | 2.0 |
| Lanette E® (Cognis) | Sodium Cetearyl Sulphate | | | | | | | | | 0.5 | | |
| Emulgin B2® (Cognis) | Ceteareth-20 | | | | | | | 0.7 | | | 1.0 | |
| **Oil Soluble UV Filters** | | | | | | | | | | | | |
| Neo Heliopan® AV (Symrise) | Ethylhexyl Methoxycinnamate | 8.0 | 8.0 | | | | 4.0 | | | 5.0 | | |
| Neo Heliopan® 303 (Symrise) | Octocrylene | | | | 5.0 | 5.0 | | | 5.0 | | 2.4 | 10.0 |
| Neo Heliopan® 357 (Symrise) | Butyl Methoxydibenzoylmethane | 4.5 | 4.5 | | 2.5 | 0.5 | 3.0 | 0.5 | 2.0 | 2.0 | 3.0 | 3.0 |
| Neo Heliopan® E 1000 (Symrise) | Isoamyl p-Methoxycinnamate | | | | 5.0 | | 4.0 | | | | | |
| Neo Heliopan® HMS (Symrise) | Homosalate | | | | 5.0 | 5.0 | | | | 3.0 | 10.0 | |
| Neo Heliopan® OS (Symrise) | Ethylhexyl Salicylate | | | | 2.0 | 5.0 | | | | 5.0 | 5.0 | 3.0 |
| Neo Heliopan® MBC (Symrise) | 4-Methylbenzylidene-camphor | | | | 1.0 | | | | | | | |
| Neo Heliopan® MA (Symrise) | Menthyl Anthranilate | | | | | 1.0 | | | | | | |
| Neo Heliopan® BB (Symrise) | Benzophenone-3 | | | | 0.5 | | 1.0 | 0.5 | 0.5 | 4.0 | 5.0 | |
| Mexoryl^{®} XL | Drometrizole Trisiloxane | | | | 1.0 | | | | | | | 3.0 |
| Tinosorb^{®} S | Bis-Ethylhexyloxyphenol Methoxyphenyl Triazine | 0.5 | 0.5 | | 0.5 | | | | | | | |
| Uvinul^{®} T-150 | Ethylhexyl Triazone | 1.0 | 1.0 | 1.0 | 0.5 | | | | | | | 0.5 |
| | -Bis[5-1(dimethylpropyl)benzoxazo l-2-yl- 1.5 5 3 (4-phenyl)imino]-6-(2-ethylhexyl)imino-1,3,5- triazine | | | | | | 1.0 | | | | | |
| Uvinul^{®} A Plus | Diethylamino Hydroxybenzoyl Hexyl Benzoate | 1.0 | 1.0 | 2.0 | 0.5 | 2.0 | | | | | | |
| Indanylidene derivatives according to DE 10055940 | | | | | 0.5 | 0.5 | | | | | | |
| Benzoylcinnamylnitrile derivatives according to WO 2006/015954 | | | | 2.0 | 0.5 | 0.5 | | | | | | |
| Parsol^{®} SLX | Polysiloxane-15 | | | | 2.0 | | | | | | | |
| Uvasorb^{®} HEB | Diethylhexyl Butamido Triazone | | | | 0.5 | | 2.0 | | | | | |
| Benzylidene butyrolactones according to EP 1008593 | | | | | 1.0 | 2.0 | | | | | | |
| Benzylidenedicarbonyl compounds described in WO2005/107692 | | | | | 0.5 | | | | | | | |
| **Water Soluble UV Filters** | | | | | | | | | | | | |
| Neo Heliopan® AP (Symrise) | Disodium Phenyldibenzimidazoletetrasulphonate | 0.5 | 0.75 | 1.0 | 0.2 | 1.5 | 0.5 | 1.0 | 0.5 | 0.3 | 2.0 | 0.5 |
| Neo Heliopan® Hydro (Symrise) | Phenylbenzimidazole- sulphonic Acid | 1.0 | 1.0 | 2.0 | 1.0 | 2.0 | 1.5 | 2.0 | 2.0 | 2.0 | 1.0 | 2.0 |
| Mexoryl^{®} SX | Terephthalylidene Dicamphor Sulfonic Acid | | | | 0.5 | | 1.0 | | | | | 0.5 |
| Sulisobenzone | Benzophenone-4 | 0.5 | | | | 1.0 | | | 0.5 | 2.0 | | |
| Neutralisation base | | qs | qs | qs | qs | qs | qs | qs | qs | qs | qs | qs |
| **Microfine UV attenuating Pigments** | | | | | | | | | | | | |
| | Titanium Dioxide | 3.0 | 3.0 | 3.0 | 0.5 | | | 6.0 | 1.0 | | | 3.0 |
| | Zinc Oxide | | | | | | 3.0 | 6.0 | | | | |
| Tinosorb^{®} M | Methylene Bis-Benzotriazolyl Tetramethylbutylphenol | | | 5.0 | 0.2 | | | | | 2.0 | | |
| **Other oil soluble components** | | | | | | | | | | | | |
| PCL Liquid 100 | Cetearyl Octanoate | | | | | 3.0 | 3.0 | | | | | |
| Corapan TQ® (Symrise) | Diethylhexyl 1,6-Naphthalate | | | | | | | 3.0 | | | 3.0 | |
| Dragoxat 89 (Symrise) | Ethylhexyl Isononoate | | | | 1.0 | 3.0 | | | | | | |
| Isoadipate | Diisopropyl Adipate | | | 3.0 | 1.0 | 3.0 | | | | | | |
| Isopropyl myristate (Symrise) | Isopropyl Myristate | | | | | | 2.0 | | | 4.0 | | |
| Neutral oil (Symrise) | Caprylic/Capric Triglyceride | | | | | 2.0 | | 5.0 | | 4.0 | | |
| Isodragol (Symrise) | Triisononanoin | | | | | | 1.0 | | 6.0 | | | |
| Cetiol OE (Cognis) | Dicaprylyl Ether | 2.0 | 2.0 | | | | 2.0 | 1.0 | 3.0 | | | |
| | Dicaprylyl Carbonate | | | 2.0 | | | 2.0 | | | | | |
| | Isohexadecane | | | | | | | | | | | 3.0 |
| Paraffin oil | Mineral Oil | | | | | | | | | 4.0 | | |
| Tegosoft TN® (Goldschmidt) | C12-15 Alkyl Benzoate | 5.0 | 5.0 | 3.0 | 4.0 | 2.0 | | | 1.0 | 4.0 | 5.0 | 5.0 |
| Abil 100® (Goldschmidt) | Dimethicone | | | | 1.0 | | | | 2.0 | | 2.0 | 0.5 |
| Dow Corning® 193 Fluid(Dow corning) | Peg-12 Dimethicone | | | | | 1.0 | | | | | | |
| | Cyclopentasiloxane | | | | | | | | | | | 5.0 |
| | Cetyl Dimethicone | | | | | | | | | | 1.0 | |
| | Hydrogenated Coco-Glycerides | 1.0 | 1.0 | | | | 1.0 | 0.5 | | | | |
| | Butylene Glycol Dicaprylate/Dicaprate | 1.0 | 1.0 | 4.0 | | | 1.0 | 7.5 | | | | |
| | Dibutyl Adipate | | | | 2.0 | | | | | | | |
| | Trimethoxycaprylylsilane | | | | | | 1.0 | | | | | |
| Lanette O® (Cognis) | Cetearyl Alcohol | | | | 1.5 | | | | | | | |
| Lanette 16® (Cognis) | Cetyl Alcohol | | | | | 1.0 | | 1.0 | | 0.5 | 1.0 | |
| Lanette 18® (Cognis) | Stearyl Alcohol | 1.0 | 1.0 | 2.0 | | | 1.0 | | 4.5 | | | |
| alpha-Bisabolol (Symrise) | Bisabolol | | | | 0.2 | 0.1 | | | | | | |
| Copherol 1250® (Cognis) | Tocopheryl Acetate | 0.5 | 0.5 | 0.5 | 0.5 | 0.5 | 0.5 | 0.5 | | 0.5 | | 0.5 |
| D-Panthenol (BASF) | Panthenol | | | 0.5 | | | | 0.5 | | 0.5 | | |
| | Retinyl-Palmitate | | | | | | 0.5 | | | | | |
| Frescolat® ML | Menthyl Lactate | | | | 0.5 | | | | 0.5 | | | |
| Fragrance | Fragrance/Parfum | qs | qs | qs | qs | qs | qs | qs | qs | qs | | |
| | Creatinine | 0.05 | 0.05 | | | | | | | | | |
| EDTA BD® (BASF) | Disodium-EDTA | 0.2 | 0.2 | 0.15 | 0.15 | 0.15 | 0.2 | 0.15 | 0.15 | 0.15 | 0.2 | 0.2 |
| **Viscosity modifiers/stability aids** | | | | | | | | | | | | |
| Bentone Gel® M IO V (Elementis Specialties) | Mineral Oil and Quaternium-Disteardimonium Hectorite and Propylene Carbonate | | | | | | | | 0.5 | | | |
| Carbopol Ulrez 10 (Noveon) | Carbomer | | | | | 0.10 | | | | 0.2 | | |
| Carbopol ETD 2001 (Noveon) | Carbomer | | | | 0.5 | | 0.1 | | | | | |
| Keltrol T® (Calgon) | Xanthan Gum | 0.2 | 0.2 | 0.3 | 0.3 | 0.4 | 0.2 | 0.2 | | | 0.2 | 0.2 |
| Pemulen TR 2 (Novion) | Acrylates/C10-30 Alkyl Acrylate Crosspolymer | | | | | | 0.2 | | 0.1 | | | 0.1 |
| Veegum ultra® (Vanderbilt) | Magnesium Aluminium Sulphate | 1.0 | 1.0 | | | 0.2 | 1.0 | | | | | |
| Aerosil^{®} 200 | Silica | | | | | | | | | 0.5 | 0.2 | |
| **Film Forming Polymers** | | | | | | | | | | | | |
| Antaron V-216/516 | VP/Hexadecene Copolymer | 0.5 | 0.5 | | | 2.0 | 0.5 | | | 1.0 | 1.0 | 2.0 |
| Antaron V-220 | VP/Eicosene Copolymer | | | | | | | | 2.0 | | | |
| Dermacryl 79 | Acrylates/Octylacrylamide Coploymer | | | | 2.0 | | 0.5 | | | | 1.0 | |
| Antaron WP-660 | Tricantonyl PVP | | 1.0 | | | | | 2.0 | | | | |
| Avalure UR 450/ 525 | PPG-17/IPDI/DMPA copolymer | | | 1.0 | | | 1.0 | | | | | |
| **Other water soluble components** | | | | | | | | | | | | |
| Water | Water (Aqua) | qs | qs | qs | qs | qs | qs | qs | qs | qs | qs | qs |
| Neutralisation base for acidic components such as carbomers, and/or stearic acid etc | | qs | qs | qs | qs | qs | qs | | qs | qs | qs | qs |
| Troxerutin | Troxerutin | 0.1 | 0.3 | 0.5 | 0.1 | 0.5 | 0.2 | 0.2 | 0.2 | 0.1 | 0.7 | 0.2 |
| Preservation agents | | qs | qs | qs | qs | qs | qs | qs | qs | qs | qs | qs |
| 1,3-Butylene glycol | 1,3-Butylene Glycol | | | | | 1.0 | | | | | | |
| Ethanol (96 %) | Ethyl Alcohol | 3.0 | 3.0 | | | | 3.0 | 3.0 | 2.0 | | | |
| Glycerin 99 % | Glycerin | 5.0 | 5.0 | 4.5 | | 0 | 5.0 | 3.0 | 5.0 | 3.0 | 3.0 | 4.0 |
| Hydrolite-5 (Symrise) | Pentylene Glycol | 5.0 | 5.0 | 3.0 | 3.0 | 2.0 | 5.0 | 4.0 | 3.0 | | | |
| Symdiol 68 | 1,2-hexylenediol and 1,2-Caprylyldiol | | | | 0.5 | | | | | | | |
| 1,2-Propylene glycol | Propylene Glycol | | | | | 1.0 | | | | 5.0 | | 5.0 |
| Soja extract | Glycine soja (soybean) germ extract | | | | 0.5 | | | | | 1.0 | 2.0 | 0.5 |
| | Sodium Ascorbyl Phosphate | | | | 0.2 | | | | | | | |
| DHA | Dihydroxyacetone | | | | | 3.0 | | | 5.0 | | | |
| Water soluble dyestuff | | Qs | qs | qs | qs | qs | qs | qs | qs | qs | qs | qs |
| Plant Extract(s) | | qs | qs | 5.00 | qs | qs | qs | qs | qs | 5.0 | qs | qs |

### 2. W/O Emulsions:, SPF > 20

| **RAW MATERIAL NAME (MANUFACTURER)** | INCI | | | | | | | | | | |
|---|---|---|---|---|---|---|---|---|---|---|---|
| | | **1** | **2** | **3** | **4** | **5** | **6** | **7** | **8** | **9** | **10** |
| **Emulsifier** | | | | | | | | | | | |
| | Polyglyceryl 2-Dipolyhydroxystearate | 4.0 | 5.0 | | | | | | | 3.0 | 2.5 |
| | PEG-45/Dodecyl Glycol Copolymer | | | | | 1.0 | | | | | |
| | Polyglyceryl 3-Polyricinoleate | | | | | | | | | 3.0 | 3.5 |
| | Cetyl PEG/PPG-10/1-Dimethicone | | | | | 1.5 | | | | | |
| | Lauryl PEG/PPG-18/18 Methicone | | | | | | 3.0 | | | | |
| | Cetearyl Alcohol, Peg-40 Castor Oil, Sodium Cetearyl Sulfate | | | | | | | 3.75 | | | |
| | PEG-30 Dipolyhydroxystearate | | | 3.5 | 3.5 | | 3.5 | 1.0 | | | |
| | Polyglyceryl-3 Methylglucose Distearate | | | | | 2.0 | | | 2.0 | | |
| | Sorbitan Stearate | | | | | | | | 1.0 | | |
| **Oil Soluble UV Filters** | | | | | | | | | | | |
| Neo Heliopan® AV (Symrise) | Ethylhexyl Methoxycinnamate | 8.0 | 2.0 | | 2.0 | 3.0 | | | | | 5.0 |
| Neo Heliopan® 303 (Symrise) | Octocrylene | 5.0 | 2.0 | 3.0 | 3.0 | 3.0 | 8.0 | | 5.0 | 10.0 | 3.0 |
| Neo Heliopan® 357 (Symrise) | Butyl Methoxydibenzoylmethane | 4.5 | 1.0 | | 2.0 | 2.0 | 3.0 | 0.5 | 2.0 | 3.0 | 3.0 |
| Neo Heliopan® E 1000 (Symrise) | Isoamyl p-Methoxycinnamate | | 1.0 | | 1.0 | 3.0 | | | | | 5.0 |
| Neo Heliopan® HMS (Symrise) | Homosalate | | 1.0 | | 1.0 | 3.0 | 2.0 | | | 3.0 | 3.0 |
| Neo Heliopan® OS (Symrise) | Ethylhexyl Salicylate | | 1.0 | | 1.0 | 3.0 | 3.0 | | | 5.0 | 5.0 |
| Neo Heliopan® MBC (Symrise) | 4-Methylbenzylidenecamphor | | 0.5 | | 0.5 | | | | | | 1.0 |
| Neo Heliopan® MA (Symrise) | Menthyl Anthranilate | | 1.0 | | 0.5 | | | | | | |
| Neo Heliopan® BB (Symrise) | Benzophenone-3 | | 1.0 | | 1.0 | | | 0.5 | 0.5 | | |
| Mexoryl^{®} XL | Drometrizole Trisiloxane | | 2.0 | | 3.0 | | 3.0 | | | | |
| Tinosorb^{®} S | Bis-Ethylhexyloxyphenol Methoxyphenyl Triazine | 1.0 | 0.5 | 2.5 | 0.5 | 3.0 | | | | 1.5 | 1.5 |
| Uvinul^{®} T-150 | Ethylhexyl Triazone | | 0.5 | 2.0 | 0.5 | 3.0 | 1.0 | | | 1.0 | 1.0 |
| | -Bis[5-1 (dimethylpropyl)benzoxazo I-2-yl-1.5 5 3 (4-phenyl)imino]-6-(2-ethylhexyl)imino-1,3,5- triazine | 0.5 | 0.5 | | 0.5 | | | | | | |
| Uvinul^{®} A Plus | Diethylamino Hydroxybenzoyl Hexylbenzoate | 1.0 | 0.5 | 2.0 | 0.5 | 0.5 | | | | 1.0 | 1.0 |
| Indanylidene derivatives according to DE 10055940 | | | 0.5 | | 0.5 | | | | | | |
| Benzoylcinnamylnitrile derivatives according to WO 2006/015954 | | | 0.5 | | 0.5 | | | | | | |
| Parsol^{®} SLX | Polysiloxane-15 | | 1.0 | | 1.0 | | | | | | 3.0 |
| Uvasorb^{®} HEB | Diethylhexyl Butamido Triazone | 0.5 | 0.5 | | 0.5 | | | | | | |
| Benzylidene butyrolactones according to EP 1008593 | | | 0.5 | | 0.5 | | | | | | |
| Benzylidenedicarbonyl compounds described in WO/2005/107692 | | | 0.5 | | 0.5 | | | | | | |
| **Water Soluble UV Filters** | | | | | | | | | | | |
| Neo Heliopan® AP (Symrise) | Disodium Phenyldibenzimidazoletetrasulphonate | 1.0 | 0.5 | 1.0 | 0.5 | 0.5 | 0.75 | 0.5 | 0.5 | 1.5 | 1.0 |
| Neo Heliopan® Hydro (Symrise) | Phenylbenzimidazole- sulphonic Acid | 0.75 | 1.3 | 2.0 | 1.0 | 1.25 | 1.5 | 2.0 | 2.0 | 1.0 | 1.0 |
| Mexoryl^{®} SX | Terephthalylidene Dicamphor Sulfonic Acid | | 0.5 | | 0.5 | | 1.0 | | | | |
| Sulisobenzone | Benzophenone-4 | 1.0 | | | | 3.0 | | | | | |
| Neutralization base | | qs | qs | qs | Qs | qs | qs | qs | qs | qs | qs |
| **Microfine UV attenuating Pigments** | | | | | | | | | | | |
| | Titanium Dioxide | 3.0 | 3.0 | 3.0 | 3.0 | 3.0 | | 6.0 | 1.0 | 3.0 | 3.0 |
| | Zinc Oxide | | | | | | | 6.0 | | | |
| Tinosorb M | Methylene BisBenzotriazolyl Tetramethylbutylphenol | | 1.0 | | 1.0 | | | | | | 3.0 |
| **Other oil soluble components** | | | | | | | | | | | |
| PCL Liquid 100 | Cetearyl Octanoate | | | | | | | | | | |
| Corapan TQ® (Symrise) | Diethylhexyl 1,6-Naphthalate | | | | | | | 3.0 | | | |
| Dragoxat 89 (Symrise) | Ethylhexyl Isononoate | | | | 1.0 | | | | | | |
| Isoadipate | Diisopropyl Adipate | | | 3.0 | 5.0 | 5.0 | | | | | |
| Isopropyl myristate (Symrise) | Isopropyl Myristate | 3.0 | | | | | 2.0 | | | 4.0 | |
| Neutral oil (Symrise) | Caprylic/Capric Triglyceride | | 5.0 | | 3.0 | | | 5.0 | | 4.0 | 4.0 |
| Isodragol (Symrise) | Triisononanoin | | | | | | | | 6.0 | | |
| | Isohexadecane | | | | | | 6.0 | | | | |
| | Dicaprylyl Carbonate | | | 5.0 | | | 8.0 | | | | |
| Cetiol OE (Cognis) | Dicaprylyl Ether | | 5.0 | | 5.0 | | | 1.0 | 3.0 | | |
| Paraffin oil | Mineral Oil | | | | | | | | | | |
| Tegosoft TN® (Goldschmidt) | C12-15 Alkyl Benzoate | 10.0 | 10.0 | 10.0 | 4.0 | 9.0 | | | 1.0 | 5.0 | 5.0 |
| Abil 100® (Goldschmidt) | Dimethicone | | 1.0 | | 1.0 | | | | 2.0 | | |
| Dow Corning^{®} 193 Fluid (Dow corning) | PEG-12 Dimethicone | | | | | | 1.0 | | | | |
| | Cetyl Dimethicone | | | | 2.0 | | | | | 2.0 | 2.0 |
| | Cyclomethicone | | | | | 15 | | | | | |
| | Cyclohexasiloxane | | | | | | 5.0 | | | | |
| | Cyclopentasiloxane | | | | | | 5.0 | | | | |
| | Simethicone | | | | | | | | | 2.0 | 2.0 |
| | Hydrogenated Coco-Glycerides | | | | 1.0 | | | 0.5 | | | |
| | Butylene Glycol Dicaprylate/Dicaprate | 7.5 | 3.0 | | 3.0 | 8.0 | | 7.5 | | | |
| | Trimethoxycaprylylsilane | | | | | 0.2 | | | | | |
| Lanette 16® (Cognis) | Cetyl Alcohol | | | | | | | 1.0 | | 0.5 | 0.5 |
| Lanette 18 ® (Cognis) | Stearyl Alcohol | | | | | | | | 3.0 | | |
| alpha-Bisabolol (Symrise) | Bisabolol | | 0.2 | | 0.2 | 0.2 | | | | 0.1 | 0.1 |
| Copherol 1250® (Cognis) | Tocopheryl Acetate | 0.5 | 0.5 | 0.5 | 0.5 | 0.5 | 0.5 | 0.5 | | 0.5 | 0.5 |
| D-Panthenol (BASF) | Panthenol | 0.5 | 0.5 | 0.5 | 0.5 | 0.5 | 0.5 | 0.5 | | 0.5 | 0.5 |
| | Retinyl Palmitate | | | 0.5 | | | 0.5 | | | | |
| Fragrance | Fragrance/Parfum | qs | qs | qs | qs | qs | qs | qs | qs | qs | qs |
| | Creatinine | 0.05 | 0.05 | 0.05 | | | | | | | |
| | Taurine | | | 1.0 | | | | | | | |
| EDTA BD® (BASF) | Disodium-EDTA | 0.2 | 0.2 | 0.15 | 0.15 | 0.15 | 0.2 | 0.15 | 0.15 | 0.15 | 0.15 |
| | | | | | | | | | | | |
| **Viscosity modifiers/stability aids** | | | | | | | | | | | |
| Bentone Gel® M IO V (Elementis Specialties) | Mineral Oil and Disteardimonium Hectorite and Propylene Carbonate | | | | | | | | 0.5 | | |
| | Microcystalline Wax | | | | | | | | | 2.0 | 2.0 |
| | Beeswax | | | | 0.3 | | | | | | |
| | Tricontanyl PVP | | | | | | | | | 2.0 | 2.0 |
| Keltrol T® (Calgon) | Xanthan Gum | | | | | | | 0.2 | | | |
| Pemulen TR 2 (Novion) | Acrylates/C10-30 Alkyl Acrylate Crosspolymer | | | | 0.3 | | | | 0.1 | | |
| | Sodium Starch Octenylsuccinate | 0.5 | 0.5 | 0.4 | | | | | | | |
| Aerosil^{®} 200 | Silica | | | | | | | | | | |
| | Magnesium Sulfate | 0.3 | 0.3 | 0.3 | | | | | | | |
| | Sodium Chloride | | | | | 0.5 | 0.5 | | | | |
| **Other water soluble components** | | | | | | | | | | | |
| Water | Water (Aqua) | qs | qs | qs | qs | qs | qs | qs | qs | qs | qs |
| Neutralisation base for acidic components such as carbomers, and/or stearic acid etc | | qs | qs | qs | qs | | qs | | qs | qs | qs |
| Troxerutin | Troxerutin | 0.2 | 0.1 | 0.3 | 0.2 | 0.5 | 0.2 | 0.3 | 0.1 | 0.7 | 0.3 |
| Preservation agents | | qs | qs | qs | qs | qs | qs | qs | qs | qs | qs |
| 1,3-Butylene glycol | 1,3-Butylene Glycol | 5.0 | 5.0 | 3.0 | 3.0 | | | | | | |
| Ethanol (96 %) | Ethyl Alcohol | 2.0 | 2.0 | 2.0 | 3.0 | 3.0 | 10.0 | 3.0 | 2.0 | 4.0 | 4.0 |
| Glycerin 99 % | Glycerin | 5.0 | 5.0 | 2.0 | 2.0 | 4.0 | 5.0 | 3.0 | 5.0 | 3.0 | 3.0 |
| Hydrolite-5 (Symrise) | Pentylene Glycol | 1.0 | 1.0 | 3.0 | 3.0 | 2.0 | 2.0 | 4.0 | 3.0 | | |
| Symdiol 68 | 1,2-hexylenediol and 1,2-Caprylyldiol | | | 0.5 | 0.5 | 0.5 | | | | | |
| 1,2-Propylene glycol | Propylene Glycol | | | | | | 3.0 | | | 5.0 | 5.0 |
| Soja extract | Glycine soja (soybean) germ extract | | | 0.5 | 0.5 | | | | | | |
| | Sodium Ascorbyl Phosphate | | | 0.5 | 0.2 | 0.2 | | | | | |
| Water soluble dyestuff | | qs | qs | qs | qs | qs | qs | qs | qs | qs | qs |
| Plant Extract(s) | | qs | qs | qs | qs | qs | qs | qs | qs | 5.0 | 5.0 |

### 3. Spray / Mousse Emulsions:, SPF > 20

| **RAW MATERIAL NAME (MANUFACTURER)** | INCI | | | | | | | | | | |
|---|---|---|---|---|---|---|---|---|---|---|---|
| | | **1** | **2** | **3** | **4** | **5** | **6** | **7** | **8** | **9** | **10** |
| **Emulsifier** | | | | | | | | | | | |
| | Polyglycery 2-Dipolyhydroxystearate | 3.0 | | | | | | | | | |
| | Disodium PEG-5 Lauryl Citrate Sulfosuccinate | 2.5 | | | | | | | | | |
| | Capryl/Capramidopropyl Betaine | 0.7 | | | | | | | | | |
| | Sodium Laureth Sulfate | 0.3 | | | | | | | | | |
| Emulgin B2® (Cognis) | Ceteareth-20 | | 1.5 | 1.5 | | | | | | 2.0 | |
| | Polyester-5 | | | | 2.5 | | | | | | |
| | Sorbitan Laurate | | | | | | 2.5 | | | | |
| | Polyglycery I- 10 Laurate | | | | | | 2.0 | | | | |
| | PPG-15 Stearyl Ether | | | | | | | 4.0 | | | |
| | Polyacrylate-3 | | | | 1.0 | | | | | | |
| | Stearyl Phosphate | | | | | | | | 2.5 | | |
| | Sorbitan Stearate | | | | | | | | | | 0.5 |
| | Stearic Acid | | | | | | 1.0 | | | | 1.0 |
| | PEG 40 Stearate | | | | | | | | | | 1.0 |
| **Oil Soluble UV Filters** | | | | | | | | | | | |
| Neo Heliopan® AV (Symrise) | Ethylhexyl Methoxycinnamate | | 6.0 | | | | | | 5.0 | | 6.0 |
| Neo Heliopan® 303 (Symrise) | Octocrylene | 5.0 | | 8.0 | 10.0 | 10.0 | 5.0 | 4.0 | | 4.0 | |
| Neo Heliopan® 357 (Symrise) | Butyl Methoxydibenzoylmethane | 3.0 | 4.0 | 4.0 | 2.0 | 2.5 | 3.0 | 5.0 | | 2.0 | 3.0 |
| Neo Heliopan® E 1000 (Symrise) | Isoamyl p-Methoxycinnamate | | | | | | | | 5.0 | | |
| Neo Heliopan® HMS (Symrise) | Homosalate | | | 3.0 | 3.0 | 5.0 | 5.0 | | | 3.0 | |
| Neo Heliopan® OS (Symrise) | Ethylhexyl Salicylate | | | 3.0 | 5.0 | 5.0 | 5.0 | 4.0 | | 3.0 | |
| Neo Heliopan® MBC (Symrise) | 4-Methylbenzylidene-camphor | | | | | | | | | 0.5 | |
| Neo Heliopan® MA (Symrise) | Menthyl Anthranilate | | | 2.0 | | | | | | | |
| Neo Heliopan® BB (Symrise) | Benzophenone-3 | | | | | | | 0.5 | | | |
| Mexoryl^{®} XL | Drometrizole Trisiloxane | | | | 4.0 | 3.0 | | | | 2.0 | |
| Tinosorb^{®} S | Bis-Ethylhexyloxyphenol Methoxyphenyl Triazine | | 2.5 | 2.5 | 1.0 | 0.5 | | | 2.5 | 1.0 | 1.5 |
| Uvinul^{®} T-150 | Ethylhexyl Triazone | 1.0 | 3.0 | 1.0 | 1.0 | 0.5 | 2.0 | | 1.0 | 1.0 | |
| | -Bis[5-1(dimethylpropyl)benzoxazo l-2-yl-1.5 5 3 (4-phenyl)imino]-6-(2-ethylhexyl)imino-1,3,5-triazine | | 1.0 | 1.0 | | | | | | 1.0 | |
| Uvinul^{®} A Plus | Diethylamino Hydroxybenzoyl Hexyl Benzoate | | 1.0 | 1.0 | | 0.5 | 1.0 | 0.5 | | 1.0 | |
| Indanylidene derivatives according to DE 10055940 | | | | | | | | | | 0.5 | |
| Benzoylcinnamylnitrile derivatives according to WO 2006/015954 | | | | | | | | | | 1.0 | |
| Parsol^{®} SLX | Polysiloxane-15 | 3.0 | | 2.0 | | 2.0 | | | | 1.0 | |
| Uvasorb^{®} HEB | Diethylhexyl Butamido Triazone | | | 1.0 | | 0.5 | | 1.0 | | 0.5 | 1.0 |
| Benzylidene butyrolactones according to EP 1008593 | | | | | | | | | | 1.0 | |
| Benzylidenedicarbonyl compounds described in WO 2005/107692 | | | | | | | | | | 1.0 | |
| **Water Soluble UV Filters** | | | | | | | | | | | |
| Neo Heliopan® AP (Symrise) | Disodium Phenyldibenzimidazoletetrasulphonate | 0.5 | 1.5 | 1.5 | 0.75 | 0.5 | 1.0 | 2.2 | 2.0 | 1.0 | 0.75 |
| Neo Heliopan® Hydro (Symrise) | Phenylbenzimidazole- sulphonic Acid | 2.0 | 2.75 | 2.50 | 2.25 | 2.0 | 2.0 | 1.5 | 1.0 | 0.5 | 2.0 |
| Mexoryl^{®} SX | Terephthalylidene Dicamphor Sulfonic Acid | | | | 1.0 | 0.5 | | | | 0.5 | |
| Sulisobenzone | Benzophenone-4 | 0.75 | 0.75 | 0.75 | 0.75 | 0.75 | 0.75 | 0.75 | 0.75 | 0.75 | 0.75 |
| Neutralisation base | | qs | qs | qs | qs | qs | qs | qs | qs | qs | qs |
| **Microfine UV attenuating Pigments** | | | | | | | | | | | |
| | Titanium Dioxide | | | | 3.0 | | | | | 1.5 | |
| | Zinc Oxide | | | | | | 3.0 | | | 1.5 | |
| Tinosorb^{®} M | Methylene Bis-Benzotriazolyl Tetramethyl-butylphenol | | 3.0 | 3.0 | | | | | | 1.0 | |
| **Other oil soluble components** | | | | | | | | | | | |
| PCL Liquid 100 | Cetearyl Octanoate | | | | | | 10.0 | | | | |
| Corapan TQ® (Symrise) | Diethylhexyl 1,6-Naphthalate | | | | | | | | 3.0 | | |
| | C18-36 Acid Triglyceride | | 1.0 | 2.0 | | | | | | 2.0 | |
| Neutral oil (Symrise) | Caprylic/Capric Triglyceride | 10 | | | | | | | 5.0 | | |
| Isodragol (Symrise) | Triisononanoin | | | | | | 2.0 | | | | |
| Cetiol OE (Cognis) | Dicaprylyl Ether | | | | | | 3.0 | | 1.0 | | |
| | Dicaprylyl Carbonate | | | 5.0 | 2.0 | | 2.0 | | 5.0 | 10.0 | |
| | Isohexadecane | | | | | | | 3.0 | | | |
| | Ethylhexylglycerin | | | | | | | | | | 0.5 |
| | Cetyl Ricinoleate | | | | | | | | | | 0.1 |
| Tegosoft TN® (Goldschmidt) | C12-15 Alkyl Benzoate | 5.0 | | | 10.0 | 8.0 | 5.0 | | 7.0 | | |
| Abil 100® (Gold-schmidt) | Dimethicone | | | | | | | | | | 4.0 |
| Dow Corning® 193 Fluid (Dow corning) | PEG-12 Dimethiconel | | | | | | 1.0 | | | | |
| | Cyclohexasiloxane | | | | | 10.0 | | | | | |
| | Cyclopentasiloxane | | | | | | | 2.0 | | | |
| | Phenyl Trimethicone | | | | | 3.0 | | 2.0 | | | |
| | Cyclomethicone | | | | | | | 1.0 | 0.5 | | |
| | Butylene Glycol Dicaprylate/Dicaprate | | 8.0 | 8.0 | | | | | 7.5 | 8.0 | 10.0 |
| Lanette 16® (Cognis) | Cetyl Alcohol | | | | | | | | 1.0 | | 0.5 |
| alpha-Bisabolol (Symrise) | Bisabolol | | 0.3 | 0.3 | | 0.2 | 0.1 | | | 0.3 | |
| Copherol 1250® (Cognis) | Tocopheryl Acetate | | 0.5 | 0.5 | 0.5 | 0.5 | 0.5 | 0.5 | 0.5 | 0.5 | |
| D-Panthenol (BASF) | Panthenol | | 0.5 | 0.5 | 0.5 | | 0.5 | | 0.5 | 0.5 | |
| | Retinyl- Palmitate | | | | | | | 0.5 | | | |
| Frescolat® ML | Menthyl Lactate | | | | | | 0.5 | | | | |
| Fragrance | Fragrance/Parfum | qs | qs | qs | qs | Qs | Qs | Qs | Qs | qs | Qs |
| | Taurine | | 1.0 | 1.0 | | | | | | 1.0 | 0.5 |
| | Creatinine | | 0.05 | 0.05 | | | | | | 0.05 | 0.05 |
| EDTA BD® (BASF) | Disodium-EDTA | 0.2 | 0.2 | 0.2 | 0.15 | 0.15 | 0.15 | 0.2 | 0.15 | 0.2 | 0.15 |
| | Sodium Chloride | 0.5 | | | | | | | | | |
| Avicel PC 611 (FMC Corporation) | Microcystalline Cellulose and Cellulose Gum | | | | | 0.80 | | | | | |
| Keltrol T® (Calgon) | Xanthan Gum | | | | | | 0.3 | | 0.2 | | |
| Pemulen TR 2 (Novion) | Acrylates/C10-30 Alkyl Acrylate Crosspolymer | | 0.2 | 0.2 | | 0.25 | | | 0.2 | 0.2 | |
| **Film Forming Polymers** | | | | | | | | | | | |
| Antaron V-216/516 | VP/Hexadecene Copolymer | | 0.5 | 0.5 | | | 2.0 | | | 0.5 | |
| Dermacryl 79 | Acrylates/Octylacrylamide Coploymer | | | | | | | 1.0 | | | |
| | Trimethylpentanediol adiopic acid glycerine copolymer | | | | | | | 1.0 | | | |
| Avalure UR 450/ 525 | PPG-17/IPDI/DMPA copolymer | | | 0.5 | | | | | | 0.5 | |
| **Other water soluble components** | | | | | | | | | | | |
| Water | Water (Aqua) | qs | qs | qs | qs | qs | Qs | Qs | Qs | Qs | Qs |
| Neutralisation base for acidic components such as carbomers, and/or stearic acid etc | | qs | qs | qs | qs | qs | Qs | Qs | | Qs | Qs |
| Troxerutin | Troxerutin | 0.3 | 0.4 | 0.6 | 0.2 | 0.2 | 0.3 | 0.4 | 0.2 | 0.4 | 0.1 |
| Preservation agents | | qs | qs | qs | qs | qs | Qs | Qs | Qs | Qs | Qs |
| 1,3-Butylene glycol | 1,3-Butylene Glycol | | | | | 3.0 | | | | | |
| Ethanol (96 %) | Ethyl Alcohol | 5.0 | 3.0 | 3.0 | 4.0 | 12.0 | | 10.0 | | 5.0 | 5.0 |
| Glycerin 99 % | Glycerin | 3.0 | 5.0 | 5.0 | 4.5 | | 5.0 | | 5.0 | 3.0 | 3.0 |
| Hydrolite-5 (Symrise) | Pentylene Glycol | | 5.0 | 5.0 | 3.0 | | | | | 3.0 | |
| Symdiol 68 | 1,2-hexylenediol and 1,2-Caprylyldiol | | | | | | | | | 0.5 | |
| 1,2-Propylene glycol | Propylene Glycol | | | | 5.0 | | 1.0 | 1.0 | 2.0 | | |
| Soja extract | Glycine soja (soybean) germ extract | | | | 1.0 | 0.5 | | | | | |
| | Sodium Ascorbyl Phosphate | | | | | 0.2 | | | | | |
| DHA | Dihydroxyacetone | | | | | | | | | | |
| Water soluble dyestuff | | qs | qs | qs | qs | qs | Qs | Qs | Qs | Qs | Qs |
| Plant Extract(s) | | qs | qs | qs | qs | qs | Qs | Qs | Qs | Qs | Qs |
| Propellant | | | | | | | | | | | Qs |

### 4. Daily Protection Preparations:

| **RAW MATERIAL NAME (MANUFACTURER)** | INCI | | | | | | | | | |
|---|---|---|---|---|---|---|---|---|---|---|
| | | **1** | **2** | **3** | **4** | **5** | **6** | **7** | **8** | **9** |
| **Emulsifier** | | | | | | | | | | |
| Emulsiphos (Symrise) | Potassium Cetyl Phosphate, Hydrogenated Palm Glycerides | 1.5 | 1.5 | 1.5 | | | | | | |
| Dracorin CE | Glyceryl Stearate Citrate | | | | | | 2.5 | | | |
| | PPG-1 Trideceth-6 | | | | | | 0.5 | | | |
| | Sorbitan Oleate | | | | | | 0.5 | | | |
| | Sucrose stearate | | | | | | | | 0.8 | |
| Hostaceron AMPS | Ammonium Polyacrylamido tauramide | | | | | | | | | 2.0 |
| | Polyglyceryl-3 Methylglucose Distearate | | | | | | | 3.5 | | |
| | Sorbitan Stearate | | | | | | | | 2.0 | |
| | Glyceryl Stearate | | | | | | | | | |
| | Isostearic Acid | 1.0 | 1.0 | 1.0 | | | | | | |
| | Stearic Acid | | | | | 2.0 | | | 1.0 | 4.0 |
| | PEG 40 Stearate | | | | | | | | | 1.0 |
| | PEG 100 Stearate | | | | 0.2 | 2.0 | | | | |
| | PEG-4 Laurate | | | | | | | | 0.3 | |
| Lanette E® (Cognis) | Sodium Cetearyl Sulphate | | | | | | | | | 0.5 |
| | Steareth-2 | | | | 0.2 | | | | | |
| | Steareth -21 | | | | 1.0 | | | | | |
| | Laureth-7 | | | | 0.75 | | | | | |
| **Oil Soluble UV Filters** | | | | | | | | | | |
| Neo Heliopan® AV (Symrise) | Ethylhexyl Methoxycinnamate | 8.0 | | | | | 4.0 | 3.0 | 5.5 | 5.0 |
| Neo Heliopan® 303 (Symrise) | Octocrylene | | 3.0 | 3.0 | 1.0 | 2.0 | | | | |
| Neo Heliopan® 357 (Symrise) | Butyl Methoxydibenzoylmethane | 2.0 | 2.0 | 2.0 | 2.0 | 2.0 | 2.0 | 1.5 | 2.0 | 1.0 |
| Neo Heliopan® HMS (Symrise) | Homosalate | | 3.0 | 3.0 | | 5.0 | | | | |
| Neo Heliopan® OS (Symrise) | Ethylhexyl Salicylate | | 3.0 | 3.0 | 4.0 | 5.0 | | | 3.0 | |
| **Water Soluble UV Filters** | | | | | | | | | | |
| Neo Heliopan® AP (Symrise) | Disodium Phenyldibenzimidazoletetrasulphonate | 0.25 | 0.5 | 0.5 | 1.0 | 1.25 | 1.0 | 1.5 | 2.0 | 0.5 |
| Neo Heliopan® Hydro (Symrise) | Phenylbenzimidazole- sulphonic Acid | 2.8 | 2.8 | 2.8 | 1.8 | 2.64 | 1.8 | 1.3 | 2.9 | 1.3 |
| Mexoryl^{®} SX | Terephthalylidene Dicamphor Sulfonic Acid | | | | | | | | | 0.5 |
| Sulisobenzone | Benzophenone-4 | 0.5 | 0.5 | 0.5 | 0.5 | 0.5 | 0.5 | 0.5 | 0.5 | 0.5 |
| Neutralizing base | | qs | qs | qs | qs | qs | qs | qs | qs | qs |
| **Microfine UV attenuating Pigments** | | | | | | | | | | |
| | Titanium Dioxide | | | | 1.0 | 1.0 | | | | |
| **Other oil soluble components** | | | | | | | | | | |
| Shea Butter | Butyrospemum Parkii | | | | | | | | | 2.0 |
| Corapan TQ® (Symrise) | Diethylhexyl 1,6-Naphthalate | | | | | | | | | |
| Dragoxat 89 (Symrise) | Ethylhexyl Isononoate | 3.0 | 3.0 | 3.0 | | 3.0 | | | | |
| Isoadipate | Diisopropyl Adipate | | | | | 3.0 | | | | |
| Isopropyl myristate (Symrise) | Isopropyl Myristate | | | | 5.0 | 5.0 | | | | |
| | Tridecyl Trimellitate | | | | | | 2.0 | | | |
| | Myristyl Myristate | | | | | | | 5.0 | | |
| Neutral oil (Symrise) | Caprylic/Capric Triglyceride | | | | | | 3.0 | | 4.0 | |
| Cetiol OE (Cognis) | Dicaprylyl Ether | | | | | | 2.0 | | | |
| | Dicaprylyl Carbonate | 2.0 | 2.0 | 2.0 | | | | 3.0 | | |
| | Isohexadecane | | | | | | | | 8.0 | |
| | Ethylhexylglycerin | | | | | | 0.5 | | | |
| Paraffin oil | Mineral Oil | | | | 2.0 | | 0.5 | | | |
| Tegosoft TN® (Goldschmidt) | C12-15 Alkyl Benzoate | | | | | | | 3.0 | | |
| Abil 100® (Goldschmidt) | Dimethicone | | | | 1.0 | | | 2.0 | | 1.0 |
| Dow Corning® 193 Fluid (Dow corning) | PEG-12 Dimethicone | | | | 1.0 | 1.0 | | | | |
| | Hydrogenated Coco-Glycerides | | | | | | 1.0 | 0.5 | | |
| | Butylene Glycol Dicaprylate/Dicaprate | | | | | | | 7.5 | | |
| | Dibutyl Adipate | | | | 2.0 | | | | | |
| Lanette O® (Cognis) | Cetearyl Alcohol | | | | 1.5 | | | | | |
| Lanette 16® (Cognis) | Cetyl Alcohol | 0.5 | 0.5 | 0.5 | 0.5 | 0.5 | 0.5 | 2.0 | 0.2 | |
| Lanette 18 ® (Cognis) | Stearyl Alcohol | | | | 0.5 | 0.5 | | | 4.5 | |
| | Myristyl Alcohol | | | | | | | | 1.0 | |
| Ceramide(s) | | | | | | | | | 0.5 | |
| alpha-Bisabolol (Symrise) | Bisabolol | | | | 0.2 | 0.1 | 0.2 | 0.1 | | |
| Copherol 1250® (Cognis) | Tocopheryl Acetate | 0.5 | 0.5 | 0.5 | 0.5 | 0.5 | 0.5 | 0.5 | 0.5 | 0.5 |
| D-Panthenol (BASF) | Panthenol | 1.0 | 1.0 | 1.0 | 0.5 | 0.5 | 0.5 | | 0.5 | 0.5 |
| | Retinyl Palmitate | 0.5 | 0.5 | 0.5 | | | | | 0.5 | |
| | Ubiquinone | 0.1 | | | | | | | | |
| Frescolat® ML | Menthyl Lactate | 0.5 | | | | | | | | |
| Fragrance | Fragrance/Parfum | Qs | Qs | Qs | Qs | Qs | Qs | Qs | Qs | Qs |
| | Niacinamide | | 0.1 | | 0.5 | 0.5 | | | | |
| | Arbutin | | 0.2 | | | | | | | |
| 4-(1-Phenylethyl)1,3-benzenediol | | 0.2 | | | | 0.5 | | | | |
| | Kojic Acid | | | 0.5 | | | | | | |
| Liquorice extract | | | | | | | 0.5 | | | |
| | Glucosyl rutin + quercitrin | 0.1 | 0.1 | | | | 0.2 | | | |
| | Isoquercitrin | | | | | | 0.1 | | | |
| | Creatinine | 0.05 | | | | | | | | |
| EDTA BD® (BASF) | Disodium-EDTA | 0.2 | 0.2 | 0.2 | 0.15 | 0.15 | 0.2 | 0.15 | 0.15 | 0.15 |
| **Viscosity modifiers/stability aids** | | | | | | | | | | |
| Carbopol Ultrez-10 (Noveon) | Carbomer | 0.15 | 0.15 | 0.15 | | 0.15 | 0.1 | | | 0.2 |
| Keltrol T® (Calgon) | Xanthan Gum | 0.2 | 0.2 | 0.2 | | | | 0.2 | | |
| Pemulen TR 2 (Novion) | Acrylates/C10-30 Alkyl Acrylate Crosspolymer | | | | | | 0.2 | | | |
| Veegum ultra® (Vanderbilt) | Magnesium Alaminium Silicate | | | | | | | | | 0.2 |
| Fucogel 1000 (Solabia) | Biosaccharide Gum-1 | | | | 0.2 | | | | | |
| Givobio GMg (Seppic) | Magnesium Gluconate | | | | | 0.2 | | | | |
| Sepigel 305 (Seppic) | Polyacrylamide and C13-14 Isoparaffin and Laureth-7 | | | | | | | | 3.0 | |
| Aerosil^{®} 200 | Silica | | | | | | | | | 0.3 |
| **Other water soluble components** | | | | | | | | | | |
| Water | Water (Aqua) | Qs | Qs | Qs | Qs | Qs | Qs | Qs | Qs | Qs |
| Neutralisation base for acidic components such as carbomers, and/or stearic acid etc | | Qs | Qs | Qs | Qs | Qs | Qs | Qs | Qs | Qs |
| Troxerutin | Troxerutin | 0.3 | 0.1 | 0.2 | 0.3 | 0.4 | 0.3 | 0.5 | 0.6 | 0.5 |
| Preservation agents | | Qs | Qs | Qs | Qs | Qs | Qs | Qs | Qs | Qs |
| 1,3-Butylene glycol | 1,3-Butylene Glycol | | | | | 1.0 | | 2.0 | 1.0 | 3.0 |
| Ethanol (96 %) | Ethyl Alcohol | | | | | | | | | 3.0 |
| Glycerin 99 % | Glycerin | | | | 5.0 | 5.00 | | 6.0 | 8.0 | |
| Hydrolite-5 (Symrise) | Pentylene Glycol | 5.0 | 5.0 | 5.0 | | 1.0 | | | | |
| Symdiol 68 | 1,2-hexylenediol and 1,2-Caprylyldiol | 0.5 | 0.5 | 0.5 | 0.5 | | | | | |
| 1,2-Propylene glycol | Propylene Glycol | | | | | | 1.0 | | | |
| Soja extract | Glycine soja (soybean) germ extract | 1.0 | | | | | | | 1.0 | |
| Peptides | | 0.3 | 0.3 | | 0.3 | 0.3 | | | | |
| | Sodium PCA | | | | | | | | 0.5 | |
| | Saccharomyces Ferment | | | | | | | | 0.3 | |
| | Ascorbyl Glucoside | | | | | | | | | 0.5 |
| Sodium or Magnesium Ascorbyl Phosphate | | 0.5 | 0.5 | 0.5 | | | | | | |
| DHA | Dihydroxyacetone | | | 5.0 | | | | 5.0 | | |
| Water soluble dyestuff | | Qs | Qs | Qs | Qs | Qs | Qs | Qs | Qs | Qs |
| Plant Extract(s) | | Qs | Qs | Qs | Qs | Qs | Qs | Qs | Qs | 5.0 |

## Claims

1. Use of troxerutin to quench the fluorescence of disodium phenyl dibenzimidazole tetrasulfonic acid or any of its salts.

2. Use according to claim 1 in a cosmetic and dermatological preparation.

3. Use according to any of claims 1 or 2, wherein the total amount of disodium phenyl dibenzimidazole tetrasulfonic acid and any of its salts is 0.2 to 4 wt.- %, and the total amount of troxerutin is 0.1 to 2 wt.- %t, each based on the total weight of the preparation said substances are used in.

4. Method for quenching the fluorescence of disodium phenyl dibenzimidazole tetrasulfonic acid or any of its salts, preferably in a cosmetic and dermatological preparation, comprising the step of combining disodium phenyl dibenzimidazole tetrasulfonic acid or a salt thereof with troxerutin, wherein troxerutin is provided in an amount sufficient to quench the fluorescence of disodium phenyl dibenzimidazole tetrasulfonic acid or its respective salt.

5. Method according to claim 4, wherein the disodium phenyl dibenzimidazole tetrasulfonic acid or its salt is provided in a cosmetically or pharmaceutically acceptable carrier.

## Patentansprüche

1. Verwendung von Troxerutin zur Fluoreszenzlöschung von Dinatrium Phenyldibenzimidazoltetrasulfonsäure oder eines ihrer Salze.

2. Verwendung nach Anspruch 1, in einer kosmetischen oder dermatologischen Zubereitung.

3. Verwendung nach einem der Ansprüche 1 oder 2, wobei der Gesamtgehalt an Dinatrium Phenyldibenzimidazoltetrasulfonsäure oder eines ihrer Salze 0,2 bis 4 Gew.-% und der Gesamtgehalt an Troxerutin 0,1 bis 2 Gew.-% beträgt, jeweils berechnet auf das Gesamtgewicht der Zubereitungen, in denen die Stoffe eingesetzt warden.

4. Verfahren zur Fluoreszenzlöschung von Dinatrium Phenyldibenzimidazoltetrasulfonsäure oder eines ihrer Salze, vorzugsweise in kosmetischen oder dermatologischen Zubereitungen, umfassend den Schritt der Vermischung von Dinatrium Phenyldibenzimidazoltetrasulfonsäure oder eines ihrer Salze mit Troxerutin, wobei das Troxerutin in einer Menge bereitgestellt wird, die ausreichend ist, um die Fluoreszenz der Dinatrium Phenyldibenzimidazoltetrasulfonsäure oder eines ihrer Salze zu löschen.

5. Verfahren nach Anspruch 4, wobei die Dinatrium Phenyldibenzimidazoltetrasulfonsäure oder eines ihrer Salze in einem kosmetisch oder dermatologisch akzeptablen Träger bereitgestellt wird.

## Revendications

1. Utilisation de la troxérutine pour étancher la fluorescence d'acide tétrasulfonique de sodium phényle dibenzimidazole ou l'un de ses sels.

2. Utilisation selon la revendication 1 dans une préparation cosmétique et dermatologique.

3. Utilisation selon une des revendications 1 ou 2, dans lequel le montant total d'acide tétrasulfonique de disodium phényle dibenzimidazole ou l'un de ses sels est 0,2 à 4 pourcentage en poids et le montant total de la troxérutine est 0,1 à 2 pourcentage en poids chacun de ceci basé sur le montant total de la préparation dans lequel lesdits produits sont utilisés.

4. Méthode pour étancher la fluorescence d'acide tétrasulfonique de disodium phényle dibenzimidazole ou l'un de ses sels, de préférence dans une préparation cosmétique et dermatologique comprenant l'étape de combinaison d'acide tétrasulfonique de disodium phényle dibenzimidazole ou un sel de celui-ci avec la troxérutine dans lequel la troxérutine est fournie dans un montant suffisant pour étancher la fluorescence d'acide tétrasulfonique de disodium phényle dibenzimidazole ou son sel respectif.

5. Méthode selon la revendication 4, dans lequel l'acide tétrasulfonique de disodium phényle dibenzimidazole ou son sel est fourni dans un transporteur cosmétique et pharmaceutique acceptable.
